# EUROPEAN PATENT SPECIFICATION

(11) **EP 1 223 978 B1**
(45) Date of publication and mention of the grant of the patent: **14.01.2009**
(21) Application number: 00967482.1
(22) Date of filing: 12.10.2000
(51) Int. Cl.: A61K 39/39, A61K 39/00, A61K 39/02, A61K 9/127, A61P 31/04, A61P 31/12, A61P 35/00

(54) **ARCHAEOSOMES AS ADJUVANTS AND CARRIERS FOR ACELLULAR VACCINES TO INDUCE CYTOTOXIC T LYMPHOCYTE (CTL) RESPONSES**
ARCHAEOSOME ALS ADJUVANTIEN UND TRÄGER FÜR AZELLULÄRE IMPSTOFFE ZUR INDUKTION EINER ZYTOTOXISCHEN T-LYMPHOZYTEN (CTL) IMMUNANTWORT
ARCHEOSOMES COMME ADJUVANTS ET PORTEURS IMMUNOMODULATEURS SERVANT A FAIRE INDUIRE PAR DES VACCINS ACELLULAIRES DES REACTIONS DE LYMPHOCYTES T CYTOTOXIQUES (CTL)

(30) Priority: 12.10.1999 US 158944 P; 08.06.2000 US 209988 P
(43) Date of publication of application: 24.07.2002
(73) Proprietor: NATIONAL RESEARCH COUNCIL OF CANADA, Ottawa, Ontario K1A OR6 (CA)
(72) Inventor: SPROTT, G., Dennis, Orleans, Ontario K4A 1Z6 (CA); KRISHNAN, Lakshmi, Gloucester Ontario K1C 7M9 (CA); CONLAN, J., Wayne, Orleans, Ontario K1C 2M7 (CA); PATEL, Girishchandra, B., Nepean, Ontario K2G 5S2 (CA); WILLICK, Gordon E., Orleans, ON, K1C 7C1 (CA)
(74) Representative: Rutherford, Claire
(86) International application number: PCT/CA2000/001197
(87) International publication number: WO 2001/026683

(56) References cited:
- WO-A-97/22333
- WO-A-99/12563
- SPROTT G ET AL: "Liposomes composed of archaeobacterial polar lipids strongly stimulate both humoral and cell-mediated immune responses to an entrapped protein, in BALB/c mice." ABSTRACTS OF THE GENERAL MEETING OF THE AMERICAN SOCIETY FOR, vol. 99, 1999, page 276 XP000993213 99th General Meeting of the American Society for Microbiology;Chicago, Illinois, USA; May 30-June 3, 1999, 1999 ISSN: 1060-2011
- NOSSAL GUSTAV J V: "Host immunobiology and vaccine development." LANCET (NORTH AMERICAN EDITION), vol. 350, no. 9087, 1997, pages 1316-1319, XP002166446 ISSN: 0099-5355
- RESCIGNO M ET AL: "Coordinated events during bacteria-induced DC maturation" IMMUNOLOGY TODAY,GB,ELSEVIER PUBLICATIONS, CAMBRIDGE, vol. 20, no. 5, May 1999 (1999-05), pages 200-203, XP004167897 ISSN: 0167-5699
- GOLOVLIOV I ET AL: "Adjuvanticity of ISCOMs incorporating a T cell-reactive lipoprotein of the facultative intracellular pathogen Francisella tularensis" VACCINE,GB,BUTTERWORTH SCIENTIFIC. GUILDFORD, vol. 13, no. 3, 1995, pages 261-267, XP004057664 ISSN: 0264-410X
- KRISHNAN LAKSHMI ET AL: "Archaeosome vaccine adjuvants induce strong humoral, cell-mediated, and memory responses: Comparison to conventional liposomes and alum." INFECTION AND IMMUNITY, vol. 68, no. 1, January 2000 (2000-01), pages 54-63, XP002166447 ISSN: 0019-9567
- KRISHNAN L ET AL: "Archaeosomes induce long-term CD8 + cytotoxic T cell response to entrapped soluble protein by the exogenous cytosolic pathway, in the absence of CD4 + T cell help." JOURNAL OF IMMUNOLOGY, (2000 NOV 1) 165 (9) 5177-85. , XP002166448
- KRISHNAN L ET AL: "The potent adjuvant activity of archaeosomes correlates to the recruitment and activation of macrophages and dendritic cells in vivo." JOURNAL OF IMMUNOLOGY, (2001 FEB 1) 166 (3) 1885-93. , XP002166449

## Description

### FIELD OF THE INVENTION

This invention relates to the field of immunology, and in particular to the use of archaeosomes as immnomodulating carriers for acellular vaccine compositions and methods to protect the vaccinated host against intracellular pathogens and cancers. The invention more specifically relates to vaccine development for enhancing cytotoxic T lymphocyte immunity in the vaccinated host and for methods for treating diseases caused by intracellular pathogens such as of microbial, protozoan and viral origin, and cancer.

### BACKGROUND TO THE INVENTION

Successful vaccination depends on two key criteria: identification of relevant antigenic target(s) for the pathogen or the disease, and the ability to evoke a strong and appropriate immune response against them in the vaccinated host. Protective immunity against intracellular pathogens (e.g., HIV, *Mycobacterium tuberculosis, Chlamydia,* malaria parasite) and cancers requires the development of antigen-specific cell mediated immune responses, especially of cytotoxic T lymphocyte (CTL) responses involving participation of CD8⁺ T cells which are capable of killing infected or neoplastic host cells (Henkart, 1997). The type of immunity required to combat intracellular infectious agents can usually be readily elicited only by vaccination with live but weakened (attenuated) forms of the pathogen. However, attenuated forms are not available for many pathogens, and if available they may either cause unacceptable side effects, be contraindicated for use in the environment, or suffer from concerns of instability and reversion to virulence (Bowersock and Martin, 1999). To avoid some of these problems, a major goal of modem vaccinology is to emulate the efficacy of such live vaccines with defined, acellular (synthetic, purified, subunit or non-replicating) vaccines. Considerable progress has been made over the past decade, towards identification, purification and/or synthesis of key antigenic determinants of pathogens and tumors. However, such highly purified proteins and/or peptides are relatively weak immunogens, limiting their ability to induce a strong protective immune response. While co-administering antigens with immunostimulating adjuvants often facilitates a strong immune response, many adjuvants have undesirable side effects, such as severe inflammatory responses, and some formulations are extremely complex for incorporating antigens, precluding their use in vaccines. Indeed, the only adjuvant currently approved universally for use in humans is alum (aluminium hydroxide), which is a relatively weak potentiator of cell mediated immunity (Gupta et al., 1995). Hence, there is a critical need, and an intense world-wide effort, to develop safe and effective adjuvants for acellular vaccines for intracellular pathogens and cancers.

Immune mechanism(s) to control infectious diseases requires the induction of neutralizing antibodies (humoral immunity) and generation of cell mediated immunity (CMI), including CD4⁺ helper (Th), and CD8⁺ cytotoxic T lymphocytes (CTL). T helper cells often segregate into dichotomous cytokine secreting phenotypes: Th1 T cells secreting IFN-γ, IL-2, and lymphotoxin aid cell-mediated immunity, whereas Th2 T cells producing IL-4. IL-5, IL-6, IL-9, IL-10 and IL-13 facilitate B cell antibody production (Krishnan and Mosmann, 1998). Naive CD8⁺ T cells are stimulated when peptides from endogenously derived antigens are presented in the context of MHC class I molecules. Although this process can occur virtually in all cells, only self-peptides, or peptides derived from viral or bacterial proteins being assembled within the host cell, are presented on MHC class I. Upon activation, naïve CD8⁺ T cells differentiate into effectors and memory T cells that possess the ability to kill infected target or tumor cells. On the other hand, protein antigens from the extracellular fluids that are taken up by antigen presenting cells (APCs) through pinocytosis, or phagocytosis in the case of particulate antigens, are fragmented within endosomes. The peptides generated are presented by the APCs in the context of MHC class II molecules and stimulate CD4⁺ T cells. CD4⁺ T helper cells contribute towards control of infection primarily by producing cytokines that aid antibody responses, inflammation, macrophage activation, and CD8⁺ T cell proliferation (Krishnan and Mosmann, 1998).

Traditional vaccines formulated with protein antigens or attenuated microbes are introduced into the endosome compartment and consequently stimulate only antibody production and, to varying degrees, T helper cell responses. However, these responses cannot eliminate pathogen-infected cells or tumor cells that require a strong CTL response. With the identification of protective immunodominant proteins that can be exploited as vaccines against intracellular pathogens and tumors, there is an urgent need for efficient strategies for the induction of long-term CTL responses to such exogenous antigens. Further, there is no clinically-approved adjuvant system capable of eliciting all immune responses (Th1. Th2, and CTL), including strong memory responses.

Adjuvant systems may be classified as carrier (vehicle) systems and/or as immune modulators. Immunestimulating complexes (ISCOMs) are open, cage-like, particulate structures with an immune modulator adjuvant (Quil A, saponins) present in the formulation (Sjolander et al., 1998). Although ISCOMs show promise because they induce a Th1 response, their cost of production and the toxicity of some saponin preparations used in ISCOMs, need to be resolved to realize their potential (Bowersock and Martin, 1999). Also, ISCOMs are not readily amenable for use with soluble antigens.

Liposomes are closed lipid vesicles containing an entrapped (encapsulated) aqueous volume. The hydrophilic polar head groups of the lipids forming liposomes are oriented towards the aqueous environment present inside and outside the liposome, whereas the hydrophobic "tail" region of the lipid is sandwiched between the polar head groups and away from the aqueous environment. Liposomes can vary in size from < 50 nm to several micrometers in diameter, depending on the lipids used and the method of preparation. Methods to encapsulate materials within the aqueous compartments of liposomes, and/or to associate with the hydrophobic lipid layer, are well known to those skilled in the art. These methods are exemplified by, but not limited to, detergent dialysis, dehydration-rehydration, reverse-phase evaporation, sonication, pressure extrusion, and remote loading. Liposomes composed predominantly of ester phospholipids, with or without additional components such as a sterol, are referred to herein as conventional liposomes. Conventional liposomes have been shown to be vehicles that provide an antigenic depot, requiring the co-delivery and/or entrapment of additional, known immune modulating adjuvants such as lipid A (Richards et al., 1998), or cholera toxin (Harokopakis et al., 1998), or cytokines (Kedar et al., United States Patent # 5,919,480, issued 6 July, 1999) to modulate the immune reaction. Specifically, induction of an antigen-specific CTL response to protein or peptide antigens encapsulated in or associated with conventional liposomes required that additional adjuvants, such a lipid A (White et al., 1995) or Quil A (Lipford et al., 1994b), also be incorporated into the vesicle. Alternatively, the liposome could be coated with oligomannose (Fukasawa et al., 1998). The difficulties with these approaches include the potential toxicity of cytokines, lipid A, and the increased costs associated with the additional adjuvants and methods for their incorporation into the liposomes.

*Archaea* are considered to be distinct from eubacteria and eukaryotes, and they include aerobic, anaerobic, thermophilic, extremely thermophilic, thermoacidophilic, and extremely halophilic microorganisms. The unique polar lipids of archaeobacteria are one of the key characteristics that help distinguish *Archaea* from the other two domains. The total lipids extracted from members of the *Archaea* consist of polar lipids and from 5-20% neutral lipids. Archaeal polar lipids are composed of branched phytanyl chains of constant length which are fully saturated in many species, and are attached via ether bonds to the glycerol backbone carbons at the *sn*-2,3 positions (Kates, 1992). In contrast, conventional ester phospholipids found in other bacteria and eucarya have fatty acyl chains of variable length, which may be unsaturated, and these are attached via ester bonds to the *sn*-1,2 carbons of the glycerol. The core structures of archaeobacterial polar lipids (the poplar head groups removed by hydrolysis) consist of the standard diether lipids (2,3-di-0-phytanyl-*sn*-glycerol or archaeol) and/or the standard tetraether lipids (2,2', 3, 3'-tetra-O-dibiphytanyl-*sn*-diglycerol or caldarchaeol) and modifications thereof (Kates, 1992). Diether lipids are monopolar like the conventional ester phospholipids, whereas the tetraether lipids are bipolar. The polar head groups, attached to the *sn*-1 glycerol carbon in the diethers and to the *sn*-1 and *sn*-1' glycerol carbons in the tetraethers, can vary and may include phospho groups, glyco groups, phosphoglyco groups, polyol groups, or hydroxy groups. However, in contrast to the phosphatidylcholine conventional lipid commonly used in conventional liposome formulations, the phasphocholine head group is rarely found in archaeobacterial polar lipids.

In earlier studies, we reported that archaeosomes facilitated a strong antibody (Th2) response to entrapped protein antigens (Sprott et al., PCT International Publication No. WO97/22333, 26 June, 1997). The antibody (humoral) response was superior to that obtained with conventional liposomes and was in some instances comparable to that obtained with the potent but toxic Freund's adjuvant. However, there is no prior art indication that adjuvants that facilitate a strongTh2 response to associated protein antigens would also stimulate strong Th1 or CTL cell mediated responses. In fact the prior art indicates to the contrary. Adjuvants such as alum (Bowersock and Martin, 1999), cholera toxin (Williams et al., 1999), and conventional liposomes in the absence of additional known immune modulators (Lipford et al., 1994b; comparative data below) induce predominantly a Th2 response, while others such as ISCOMs evoke primarily Th1 (cell mediated) immunity (Sjolander et al., 1998). For peptide antigens, in many instances alum and conventional liposomes alone even fail to induce a Th2 response (White et al., 1995). Moreover, it is well known that Th1 and Th2 responses are often dichotomous (Krishnan and Mosmann, 1998).

The rationale for developing vaccines for cancer prevention or immunotherapy is that cancer cells can express specific tumor antigens, which may be used in a vaccine to raise a tumor-specific CTL response. A major obstacle to overcome is the development of antigen delivery and adjuvant systems capable of stimulating the immune system to mount a sufficiently strong, antigen-specific CTL response. The utility of archaeosomes as an immunomodulating carrier of a cellular antigens is further demonstrated for tumor antigens, using a solid tumor model in mice.

The citation of above preferences is not an admission that any of the foregoing is pertinent prior art. Representations as to the contents of these references and as to the date of publication are based upon the information available to the applicants and do not constitute any admissions as to the correctness of the contents or the dates of said references

### SUMMARY OF THE INVENTION

The invention as defined by the claims provides for use of a composition comprising a liposome prepared from a polar lipid extract of an archeobacterium and an antigen having an MHC class I restricted epitope in the manufacture of a medicament for prophylactic and/or therapeutic use against cancer and/or infection by intracellular pathogens, wherein the liposome serves as an adjuvant, and the medicament elicits an antigen-specific CD8⁺ T cell response in an animal.

Archaeobacteria useful for practice the invention may include *Thermoplasma acidophilum*, *Methanobrevibacter smithii*, *Halobacterium, salinarum* or *Natronobacterium magadii.*

Thus, the invention proposes the use of archaeosomes as immunomodulating carriers for antigens in a cellular (non-replicating) vaccine formulations, capable of inducing cytotoxic T cell responses in the immunized host.

The invention also proposes archaeosomes as immunomodulating carriers for antigens in a cellular (non-replicating) vaccine formulations, capable of inducing immune responses in the immunized host.

Archaeosomes may also be used as immunomodulating antigen carriers in vaccine formulations for prophylactic and therapeutic uses against cancer.

The invention offers the opportunity to selectively elicit an antigen specific cytotoxic T cell response in an animal, by administering to the animal a vaccine composition comprising a liposome prepared from the total polar lipids extract of an archaeobacterium and an antigen, wherein the liposome serves as an adjuvant.

The liposome may serve as an immunomodulating carrier for the antigen.

Administration to an animal of a vaccine composition comprising a liposome prepared from the total polar lipids extract of an archaeobacterium and an antigen may activate antigen presenting cells in an animal by upregulating costimulatory molecules 87.1 (CD80) and B7.2 (CD86) on the surface of the antigen presenting cells.

Simlarly, the invention, may provide for activating CD11c+ dendritic cells in an animal.

The invention may also be used for stimulating the production of cytokine interferon gamma in an animal, or the production of the cytokines IL-4 and interferon gamma in an animal, by administering to the animal a vaccine composition comprising a liposome prepared from polar lipids extract of an archaeobacterium, preferably *Methanobrevibacter smithii*, and an antigen.

Similarly, the invention may also be applied in a method for stimulating the production of tumor necrosis factor by antigen presenting cells in an animal.

The vaccines of the invention may also be applied in recruiting Macl or cells in an animal; or for stimulating T cell proliferation and cytokine production; or for eliciting an antigen specific cytotoxic T cell response in an animal.

A vaccine composition may comprise a liposome prepared from a polar lipid isolated in a biologically pure form from an archaeobacterium and an antigen.

Such a vaccine may be used for conferring to an animal protective immunity against infection by an intracellular pathogen; or for immunizing an animal to confer to the said animal a memory response against infection by an intracellular pathogen.

A vaccine of this invention, may be used for eliciting an antigen specific MHC class I-restricted cytotoxic T lymphocyte response and an antigen specific MHC classll-restricted Th 1, Th 2 response in an animal.

The vaccines provided by the invention may confer to an animal immunity against cancer.

The vaccine composition may comprise a liposome prepared from the polar lipids extract of an archaeobacterium and an antigen, wherein the antigen is an alkylated peptide.

### BRIEF DESCRIPTION OF THE DRAWINGS

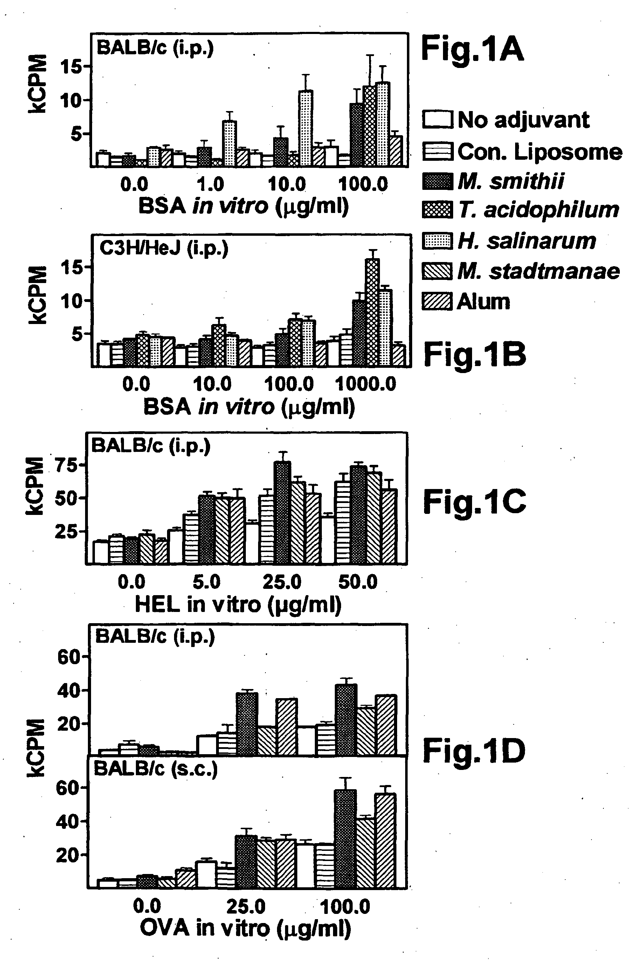

**Fig.1** shows that i.p. or s.c immunization of BALB/c or C3H/HeJ strains of mice with various archaeosomes carrying encapsulated antigen (Ag) induces development in the mice, of spleen cells that exhibit strong antigen-specific spleen cell proliferation *in vitro.* The proliferative responses (mean kCPM ± SEM of mice in each group) of spleen cells obtained from Ag-archaeosome immunized mice was superior to that of mice immunized with Ag-alone (No adjuvant) or with Ag encapsulated in conventional liposomes (Con. Liposome). Data from mice immunized with the Ag adsorbed onto alum (Alum) are shown for comparative purposes using BSA (Fig. 1A,B), HEL (Fig. 1C) or OVA (Fig. 1 D) as illustrative examples for the Ag.

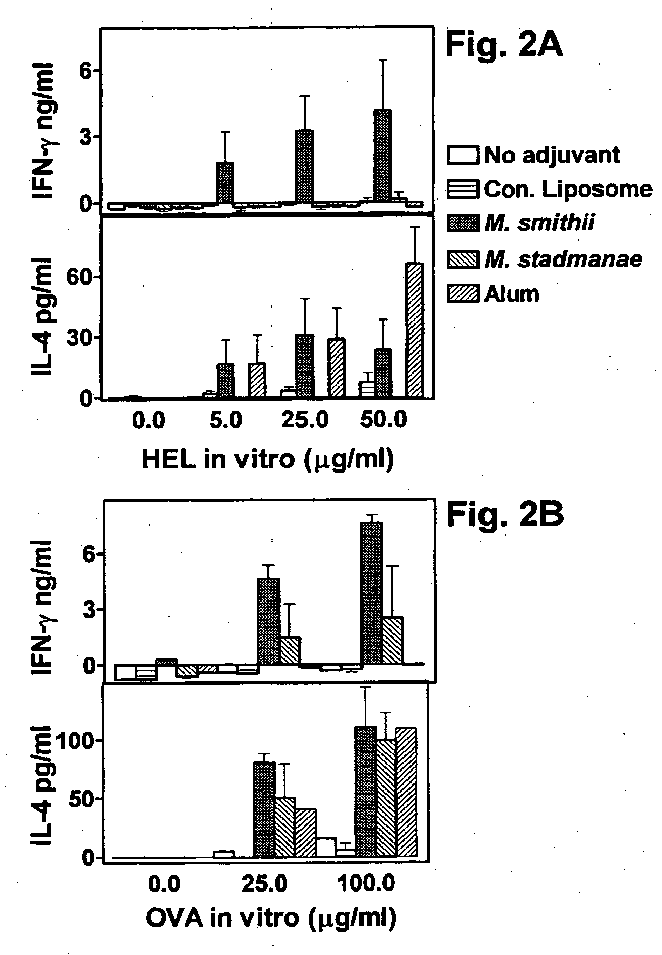

**Fig. 2** shows that spleen cells from BALB/c mice immunized (day 0 and 21) with antigen (Ag) encapsulated in the indicated archaeosomes induce production of both Th1 (IFN-γ) and Th2 (IL-4) cytokines (mean cytokine production ± SEM of mice in each group). Mice were immunized i.p. with 10 µg HEL in archaeosomes (Fig. 2A), or s.c. with 15 µg OVA in archaeosomes (Fig. 2B). Data from groups of mice immunized with antigen alone (No adjuvant), Ag encapsulated in conventional liposomes (Con. Liposome), and Ag adsorbed onto alum (Alum) are included for comparative purposes.

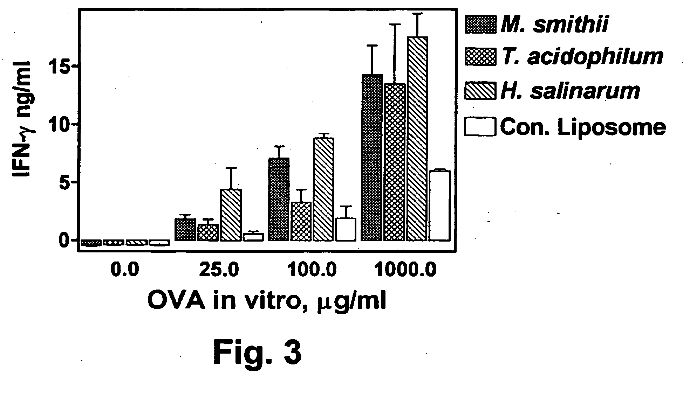

**Fig. 3** shows more examples of the superior OVA-specific induction of IFN-γ production in splenic cultures from mice immunized s.c. with OVA encapsulated in archaeosomes compared to conventional liposomes.

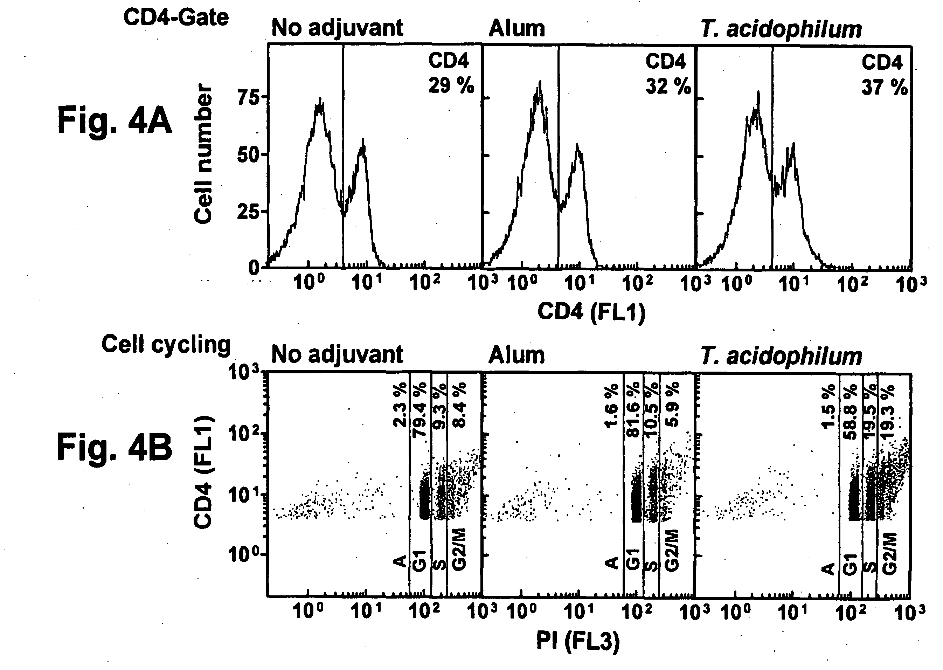

**Fig. 4** shows that upon re-exposure to Ag alone, strong memory responses are induced in mice previously immunized with Ag-archaeosomes, as illustrated by CD4⁺ T cell cycling profiles obtained on gated CD4⁺ cells. Profiles in Fig. 4A indicate the CD4⁺ (FL1) gate. Plots in Fig. 4B indicate the percentage of CD4⁺ tells in various phases of the cell cycle [based on propidium iodide (FL3) content] in each group. 'A' denotes apoptotic, `G1' resting, 'S' synthetic, and 'G2/M' mitotic phase.

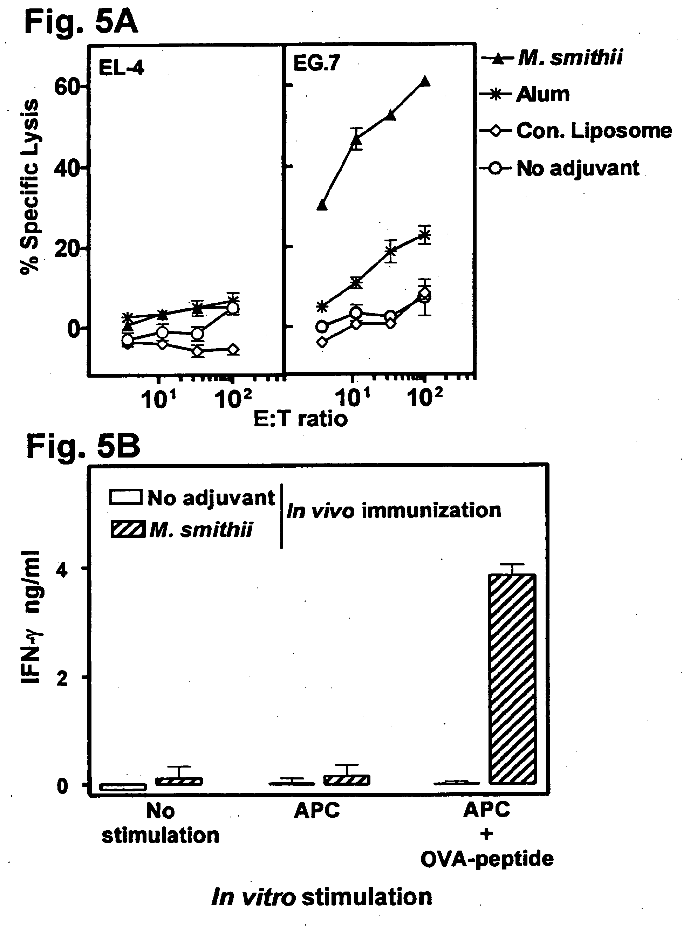

**Fig. 5** shows that the induction of splenic CTL cells following immunization of mice with OVA entrapped in *M*. *smithii* archaeosomes is superior to that in mice immunized with OVA in PBS alone (No adjuvant), or with OVA either entrapped in conventional liposomes (Con. Liposome) or adsorbed onto alum. In Fig. 5A, CTL responses are shown as % specific lysis of triplicate cultures ± SD at various Effector:Target (E:T) ratios on EL-4 (non-specific target) and EG.7 (specific target expressing OVA peptides) cells. Spleen cells from mice immunized with OVA-M. *smithii* archaeosomes were stimulated with OVA₂₅₇₋₂₆₄ peptide-pulsed-APCs (IC21 cells) and IFN-γ production (ng/ml ± SD) determined (Fig. 5B) in the absence of APC stimulation (no activation), in the presence of unloaded APCs (APC), and after stimulation with OVA peptide pulsed-APCs (APC + OVA-peptide).

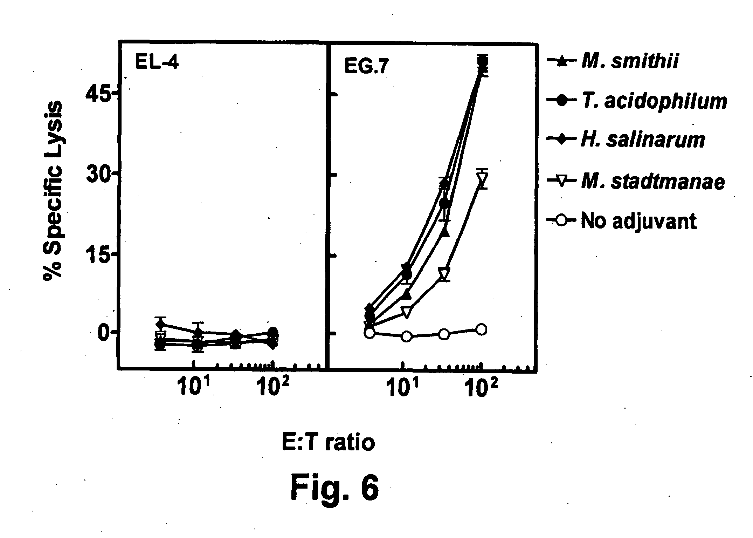

**Fig. 6** shows that a strong CTL activity (% specific lysis) is also detected in mice immunized with OVA entrapped in other archaeosomes.

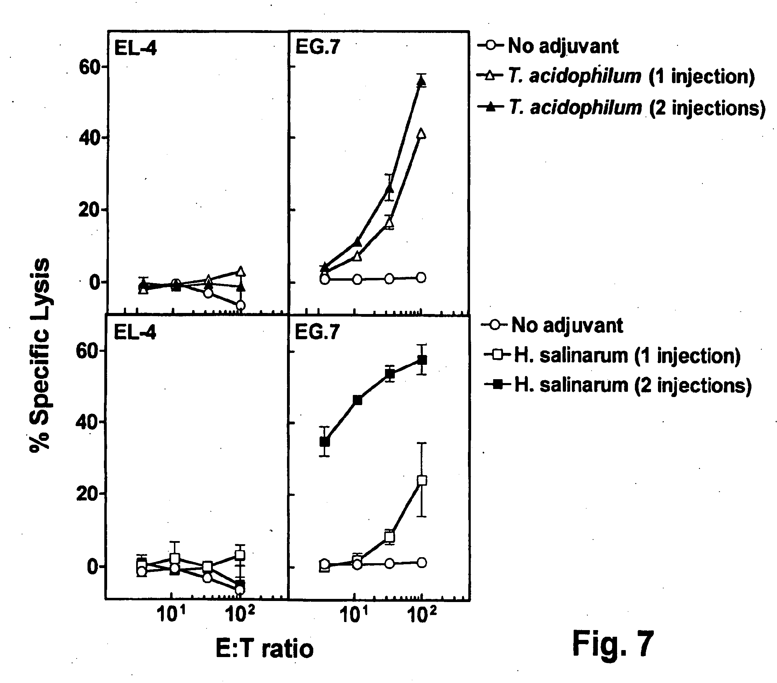

**Fig. 7** shows that the CTL activity (% specific lysis) obtained after a single injection of C57BU6 mice with OVA-archaeosomes is enhanced further upon a booster injection of the mice.

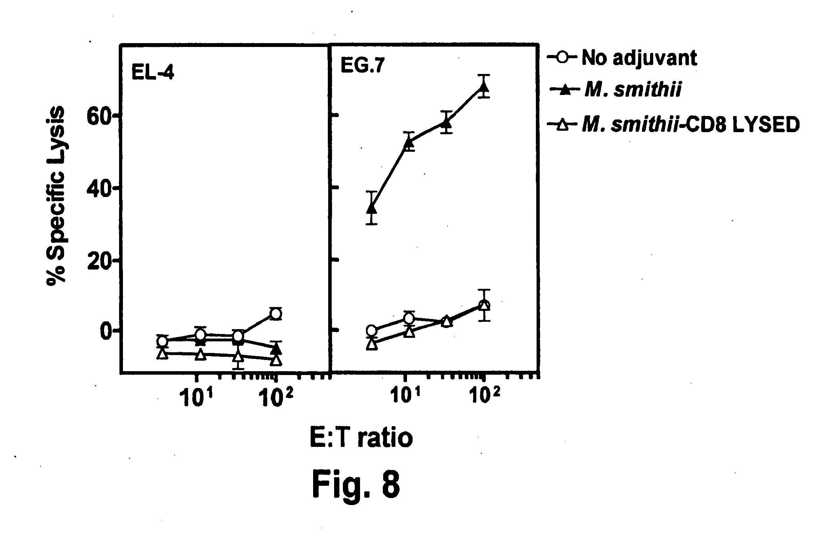

**Fig. 8** shows that the CTL activity (% specific lysis) induced in mice immunized with OVA-archaeosomes is mediated through CD8⁺ T cells. Mice were immunized with 15 µg OVA entrapped in *M. smithii* archaeosomes. Lysis of CD8⁺ T cells in spleen cell populations from archaeosome immunized mice resulted in loss of CTL activity.

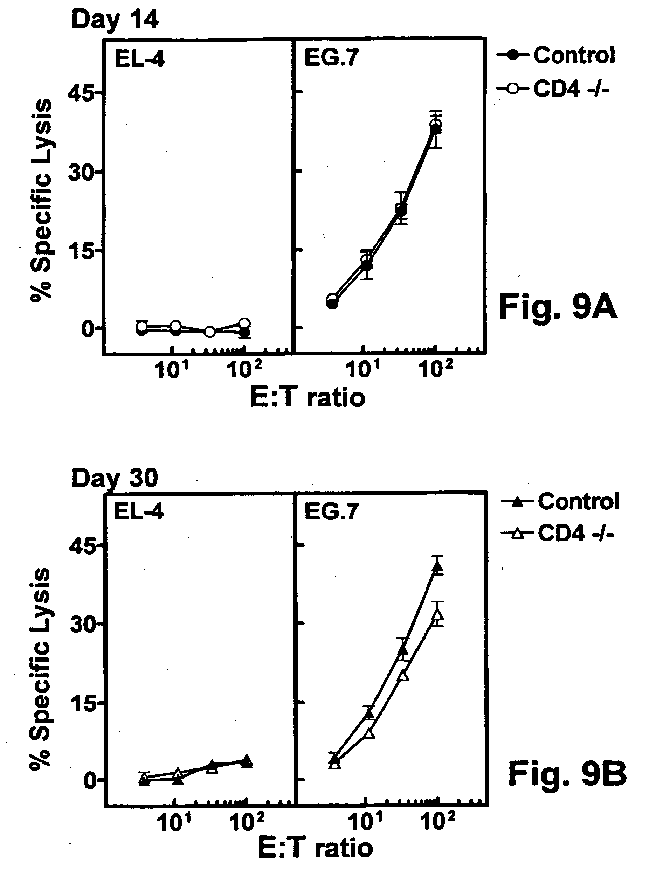

**Fig. 9** shows that immunization of CD4⁺ T cell-deficient mice with OVA-*M*. *smithii* archaeosomes results in strong CTL activity (% specific lysis ± SD of triplicate cultures) comparable to that in the normal control immunized mice, assayed on day 14 (Fig. 9A) and day 30 (Fig. 9B) after a s.c. immunization.

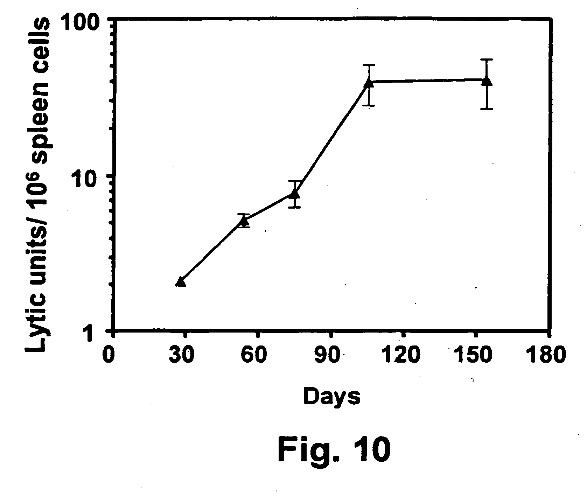

**Fig. 10** shows that immunization of C57BU6 mice with OVA-archaeosomes induces a strong and long-lasting CTL memory response to the antigen. Data are shown as lytic units ± SD for spleen cells, from individual mice.

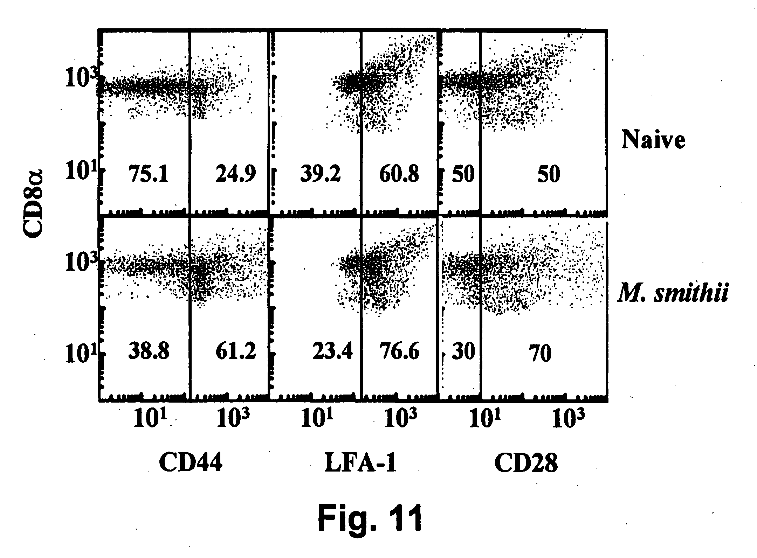

**Fig. 11** is flow cytometry data showing that archaeosomes modulate expression of cell surface molecules on splenic CD8⁺ T cells. Representative mice (n=2) from experiments described in Fig. 10 and naïve control mice (n=2) were challenged (i.p.) with OVA₂₅₇₋₂₆₄peptide-pulsed-IC21 macrophages on day 140 post-immunization. Analyses of splenic CD8⁺ T cell populations 5 days later showed that higher amounts of CD44, LFA-1 (CD11a) and CD28 proteins were expressed in splenic cells from Ag-archaeosome immunized mice compared to naïve controls. Numbers within each panel indicate the percentage of CD8⁺ T cells staining for each marker.

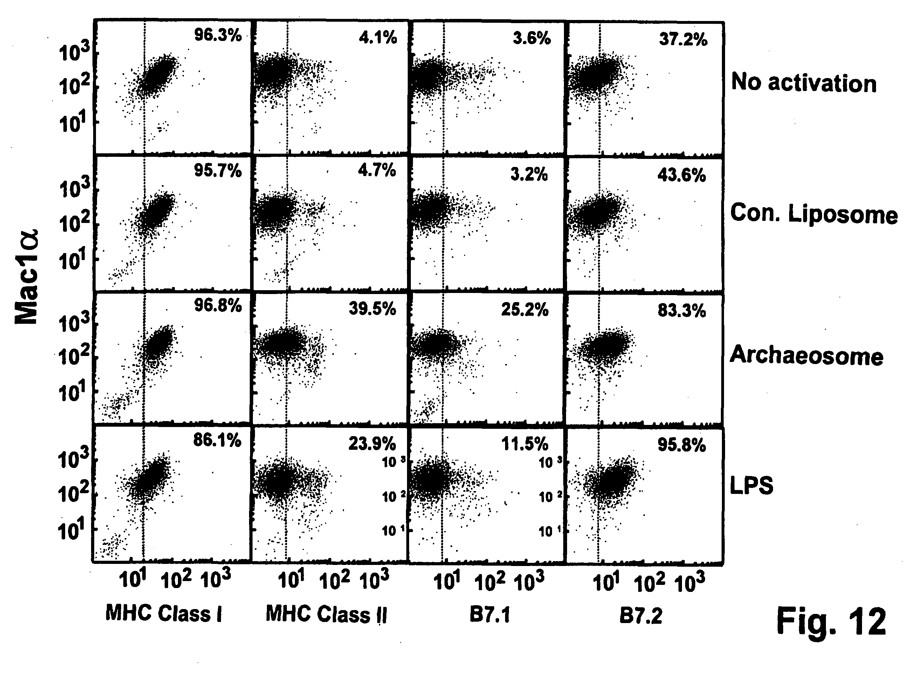

**Fig. 12** is flow cytometry data (20,000 events) showing that empty archaeosomes upregulate expression of cell surface molecules on J774A.1 cells during growth *in vitro.* The enhanced expression of MHC Class II, B7.1 and B7.2 molecules in J774A.1 macrophages treated with *M. smithii* archaeosomes (Archaeosome, 25 µg lipid/ml) was comparable or exceeded the upregulation seen in the positive control (LPS, 10 µg/ml). Effects with conventional liposomes (Con. Liposome, 25 µg lipid/ml) were minimal and comparable with those of naïve (No activation) controls. The hashed line indicates the positive stain for each activation marker. Numbers within each panel indicate the percentage of Mac 1α⁺ cells staining for each marker in the various treatment groups.

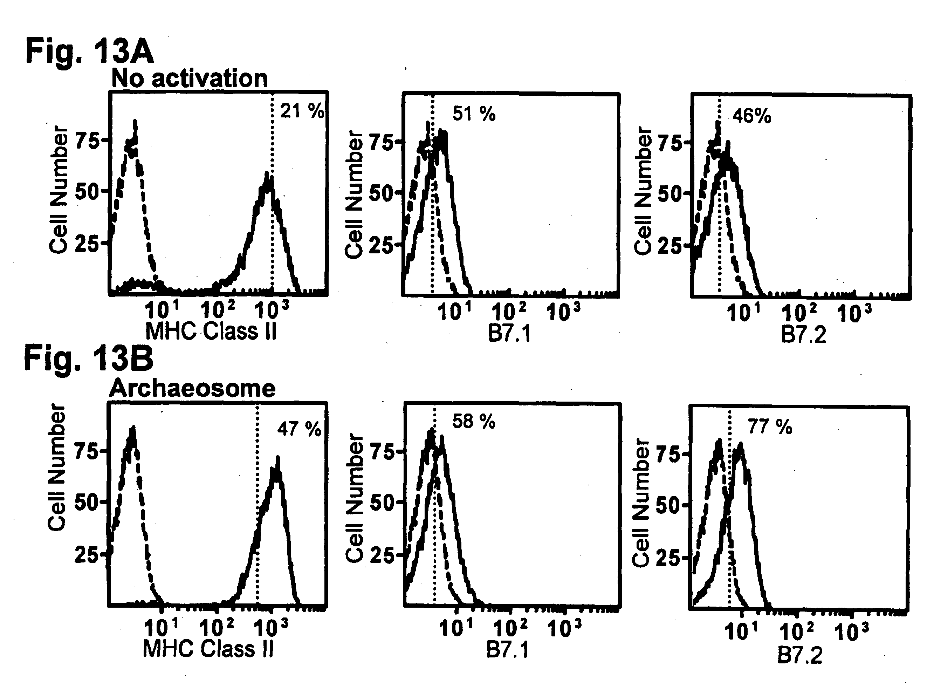

**Fig. 13** is flow cytometry data (analysis of 20,000 events) showing upregulation of cell surface molecules on bone marrow derived dendritic cells (DCs) treated with empty archaeosomes *in vitro.* Flow cytometry data were obtained on DCs (10⁵/ml) that were cultured for 24 h in the absence (i.e., No activation, Fig. 13A) or presence (Fig. 13B) of *M. smithii* archaeosomes (25 µg lipid/ml). The hashed line indicates the negative staining (with the isotype-specific antibody) and the solid line the positive staining. The percentages within each panel indicate the number of cells staining strongly for each marker in the two groups.

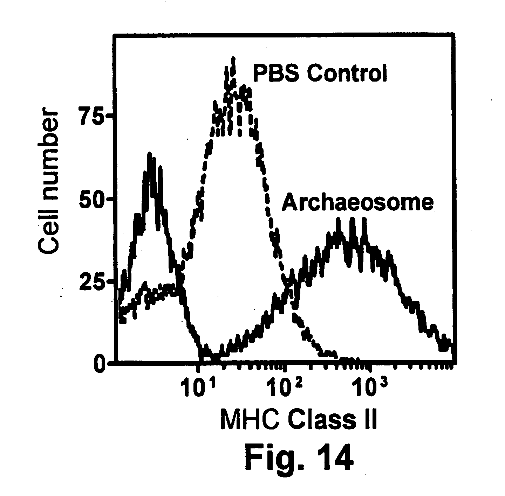

**Fig. 14** is flow cytometry data (analyses of 20,000 evenys) showing that empty archaeosomes administered i.p. into mice result in upregulation of MHC class II expression on peritoneal exudate cells. The hashed line indicates expression profile of cells from control mice treated with PBS alone, and the solid line indicates the MHC Class II enhanced expression profile of cells from *M. smithii* archaeosome-treated mice.

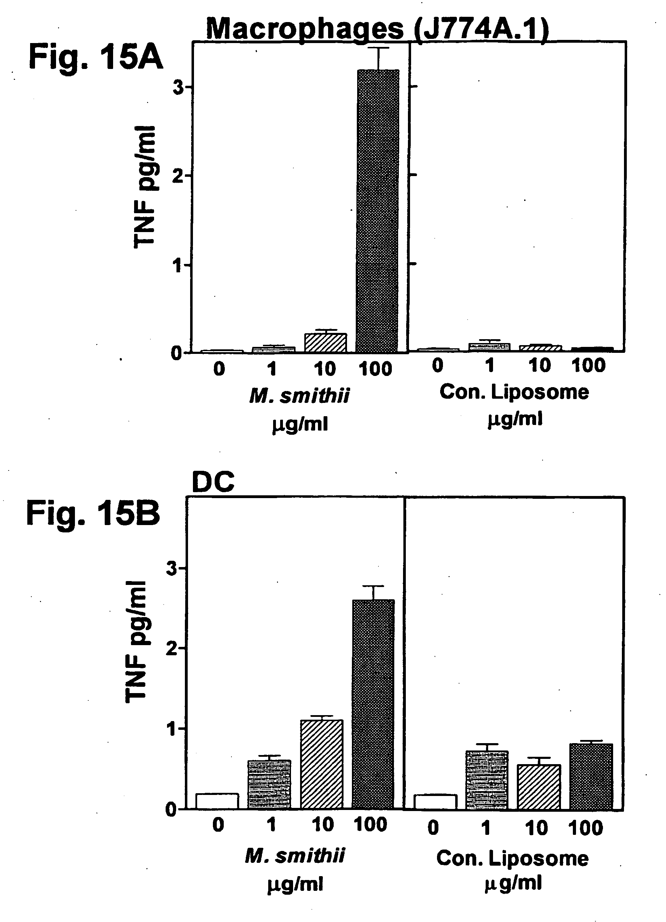

**Fig. 15** is histograms showing that compared to conventional liposomes (Con. Liposome), *M. smithii* archaeosomes induce J774A.1 macrophages (Fig. 15A) and dendritic cells (Fig. 15B) to produce substantial amounts of tumor necrosis factor-α (TNF). Macrophages or DCs were treated with varying concentrations of conventional liposomes or *M*. *smithii* archaeosomes for 48 h *in vitro,* and TNF produced (± SD of triplicate cultures) in the supernatant assessed by a bioassay. Sensitivity of the TNF assay was <0.1 pg/ml.

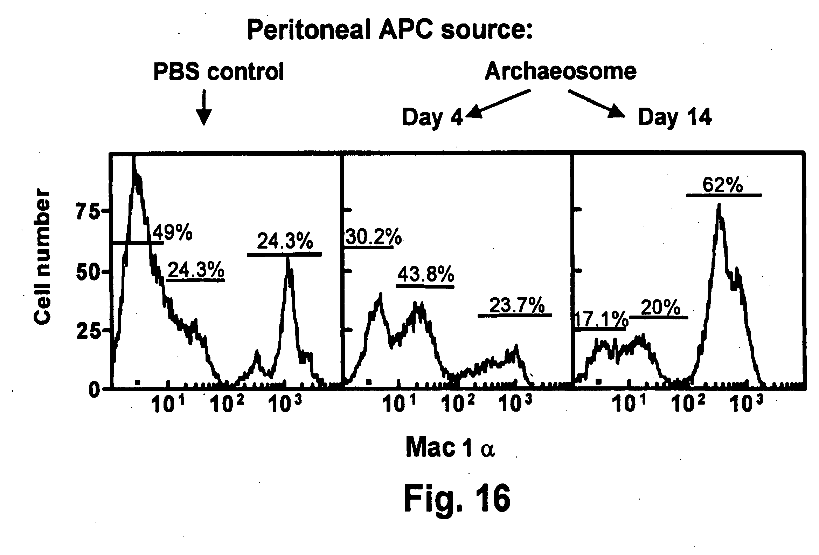

**Fig. 16** is flow cytometry data (analysis of 20,000 events in each sample) showing that empty archaeosomes injected i.p. into mice result in recruitment of Mac1 α⁺ cells into the peritoneum. BALB/c mice (n=2) were injected with 1 mg/200 µl *M. smithii* archaeosomes (Archaeosome), or 200 µl of PBS (PBS control). After 4 and 14 days, cells were recovered by peritoneal lavage, and analyzed for the expression of Mac1α by flow cytometry. PBS control mice exhibited similar cell profiles on all days post-injection. Three distinct populations were discerned based on Mac1α staining; Mac1α⁻, Mac1α^{low}, and Mac1α^{hi}, as indicated from the % cell numbers shown in the figure.

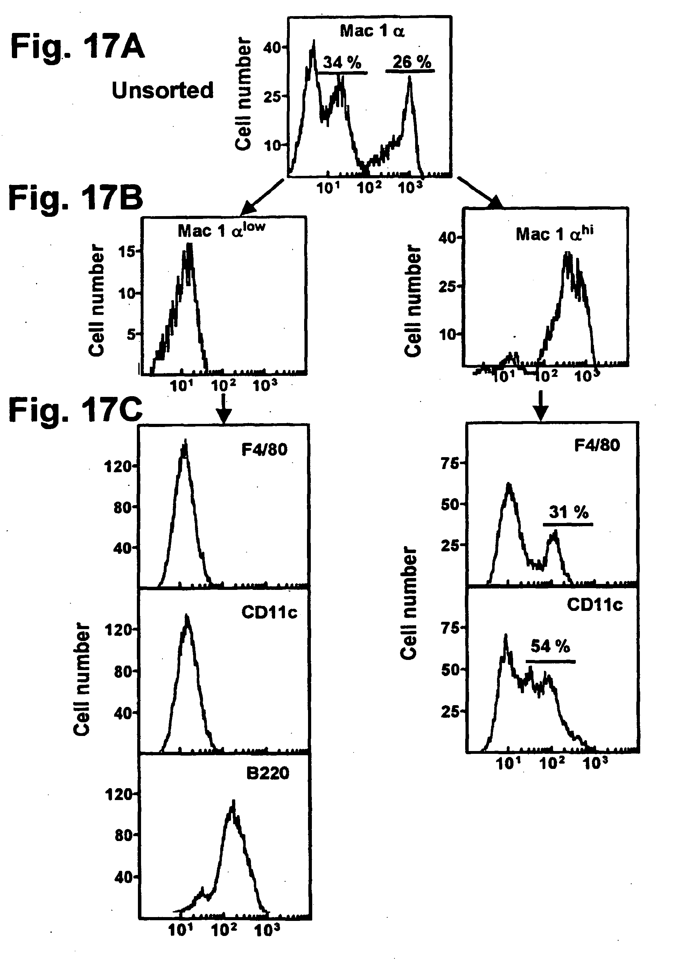

**Fig. 17** is flow cytometry data showing that the Mac 1α^{hi} population induced in the peritoneum of archaeosome-injected mice consists of macrophage (F4/80) and dendritic cells (CD11c). BALB/c mice were injected i.p. with 200 µl of PBS or 1 mg of *M. smithii* archaeosomes in 200 µl of PBS. On day 5, peritoneal exudate cells were analyzed for the expression of Mac 1 a (Fig. 17A). Based on Mac1α expression, as indicated in the figure, cells were sorted into Mac1α^{low} and Mac1α^{hi} populations (Fig. 17B). The sorted cells (2 X 10⁵/ml) were cultured *in vitro* for 48 h with GM-CSF (5 ng/ml). Cultured cells were then recovered, washed and analyzed for the expression of F4/80, CD11c, and B220 (Fig. 17C). All profiles are deduced from the analysis of 20,000 events.

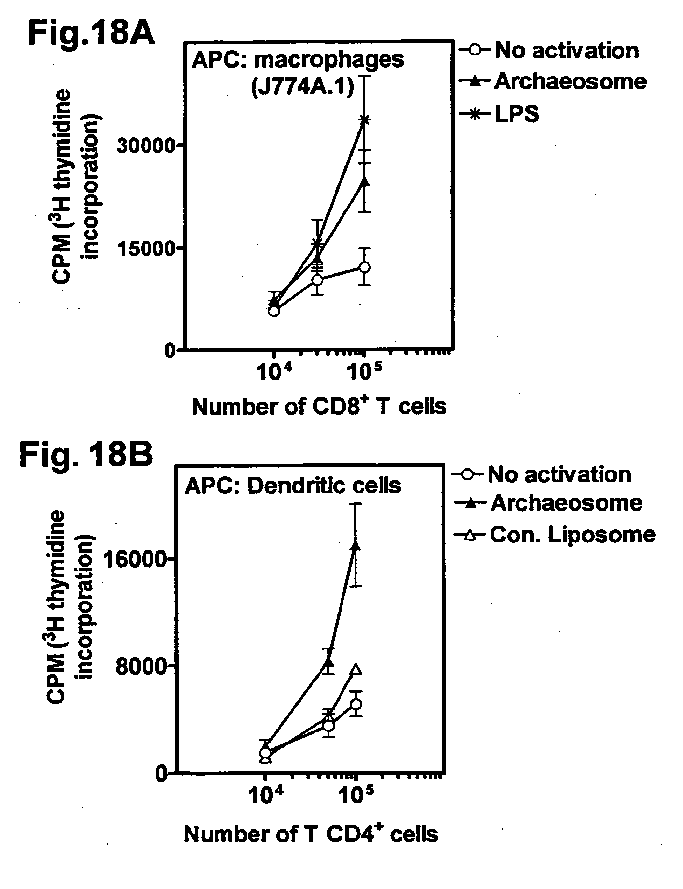

**Fig. 18** is proliferation data comparing stimulation of allo-specific T cells by APCs treated with empty *M. smithii* archaeosomes (25 µg lipid/ml) or conventional liposomes (Con. Liposome, 25 µg lipid/ml) or LPS (10 µg/ml). J774A.1 macrophages or bone marrow dendritic cells were treated with archaeosomes or LPS and the APCs were then used for stimulating purified allo-specific (H-2K^{b}) CD8⁺ T cells (Fig. 18A) or CD4⁺ T cells (Fig. 18B). Data represent the mean counts per min (CPM) ± standard deviation of triplicate cultures.

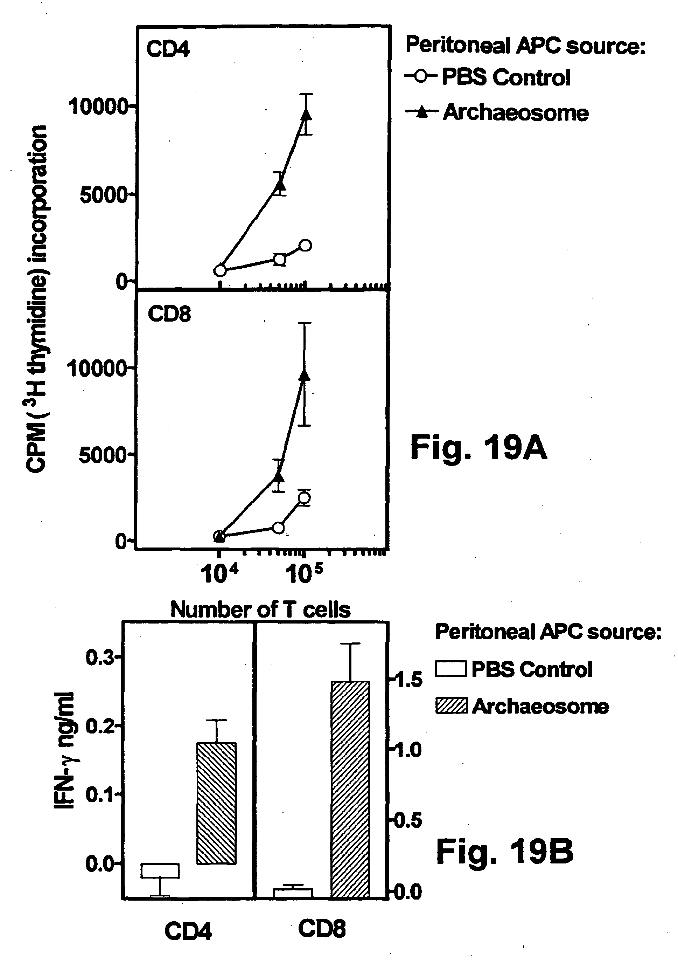

**Fig. 19** shows stimulation of allo-specific T cells by peritoneal exudate cells taken from mice treated with empty archaeosomes. BALB/c mice (n=3) were treated with archaeosomes or PBS and 5 days later the peritoneal exudate cells were recovered. These cells were used as APCs to stimulate allo-specific (H-2K^{b}) purified CD4 and CD8⁺ T cells. The proliferation of the T cells was monitored by ³H incorporation at 72 h (Fig. 19A). IFN-γ was measured in culture supernatants by ELISA (Fig.19B). Data represent mean ± SD of values from triplicate cultures.

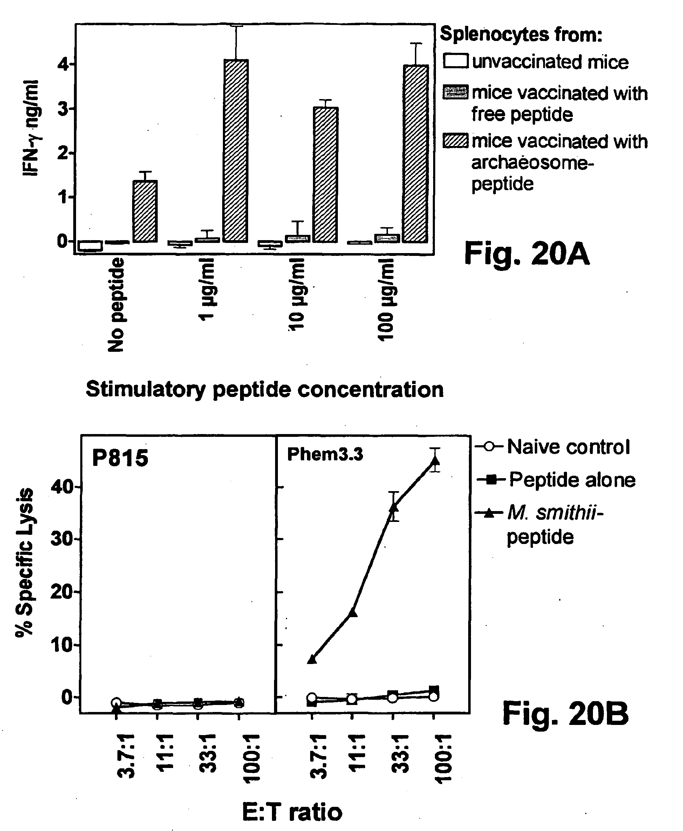

**Fig. 20** shows that splenocytes from mice immunized with a peptide-archaeosome vaccine exhibit antigen-specific stimulation of IFN-γ (Fig. 20A) and CTL activity (Fig. 20B). CTL activity is shown as % specific lysis ± SD for triplicate cultures using a range of effector to target ratios (E:T).

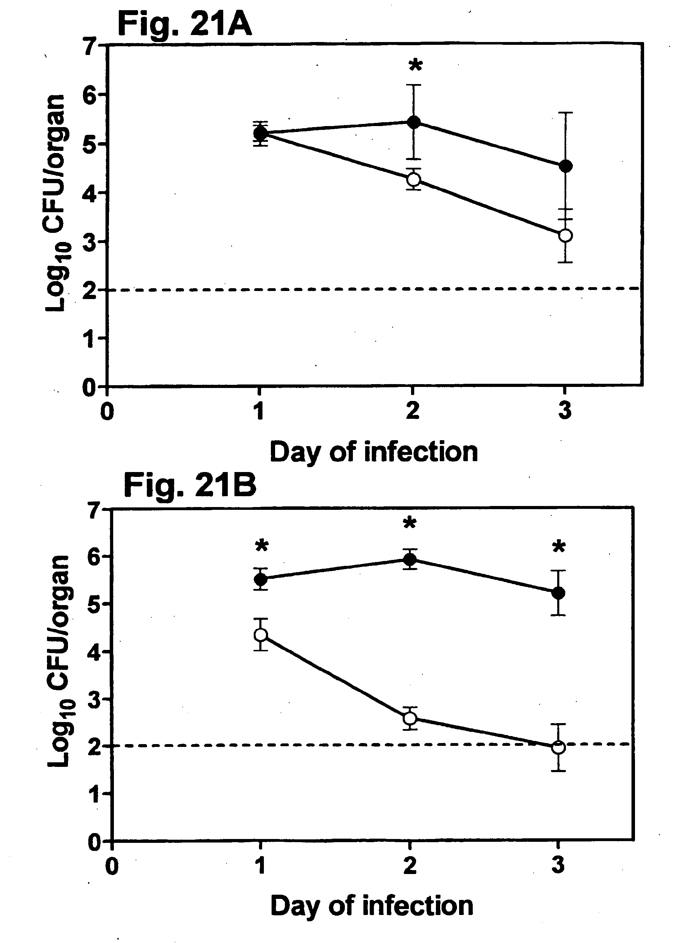

**Fig. 21** is a time course showing the kinetics of expression of anti-*Listeria* immunity in BALB/c mice vaccinated with 20-mer lipopeptide encapsulated in archaeosomes. Mice were vaccinated with 12.5 µg of the 20-mer lipopeptide antigen encapsulated in *M. smithii* archaeosomes (antigen in 0.5 mg archaeosomes in 100 µl PBS) (o), or with 100 µl PBS alone (•). Six weeks after the last vaccination, mice were challenged with *L. monocytogenes.* Bacterial burdens were determined in the livers (Fig. 21A), and spleens (Fig. 21 B) of vaccinated and control mice 1, 2, and 3 days after challenge.* , indicate bacterial burden significantly lower (p<0.05) in immunized versus control mice according to the Mann Whitney rank sum test. Broken line, lower limit of bacterial detection.

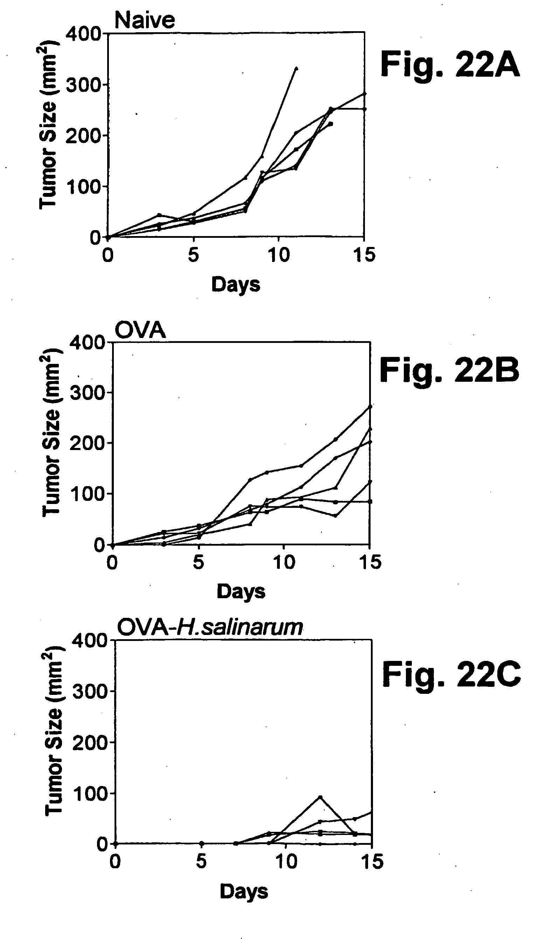

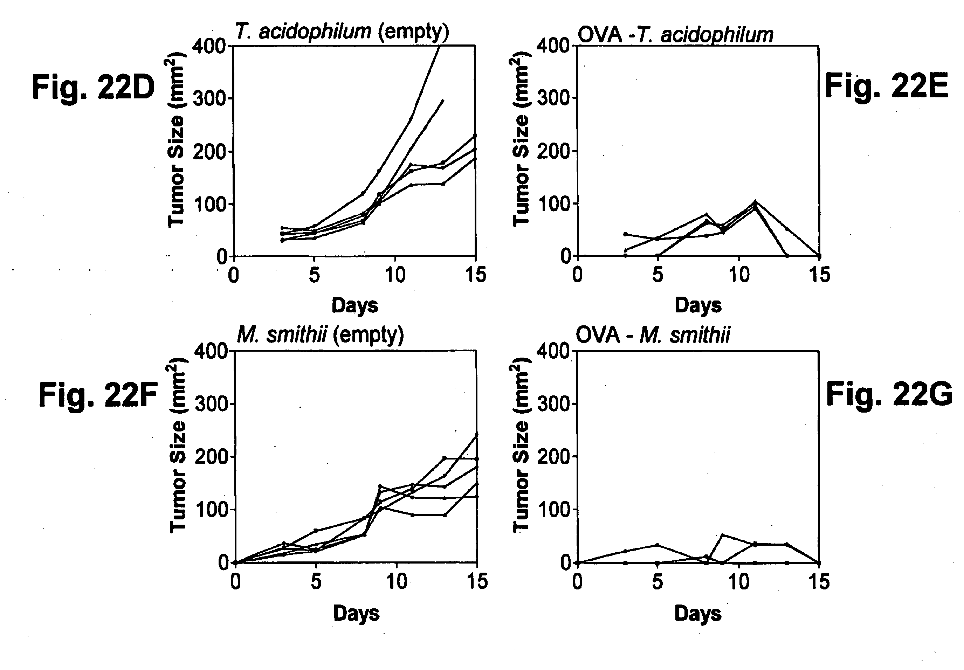

**Fig. 22** shows that immunization of mice with antigen encapsulated in archaeosomes provides protection against growth of solid tumors. C57BL/6 mice were immunized on days 0 and 21, with nothing (i.e., naïve mice, Fig. 22A), 15 µg OVA in PBS alone (Fig. 22B), with 15 µg OVA encapsulated in *H*. *salinarum* (Fig. 22C), *T*. *acidophilum* (Fig. 22E), or *M. smithii* (Fig. 22G) archaeosomes, or with empty *T*. *acidophilum* (Fig. 22D) or *M. smithii* archaeosomes (Fig. 22F). Mice were challenged 8 weeks post first iimmunization with 10 million EG.7 (T cell lymphoma cells transfected with gene encoding OVA) tumor cells.

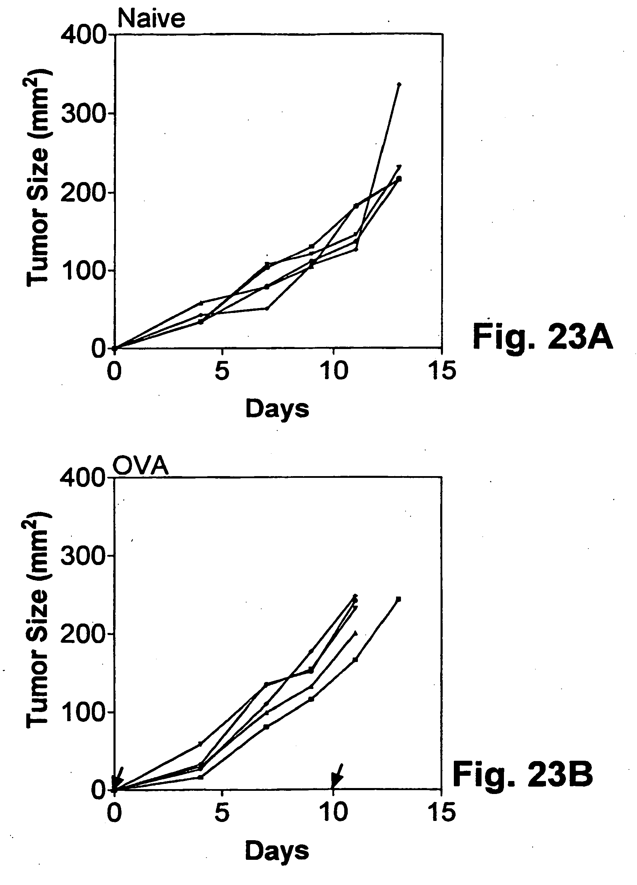

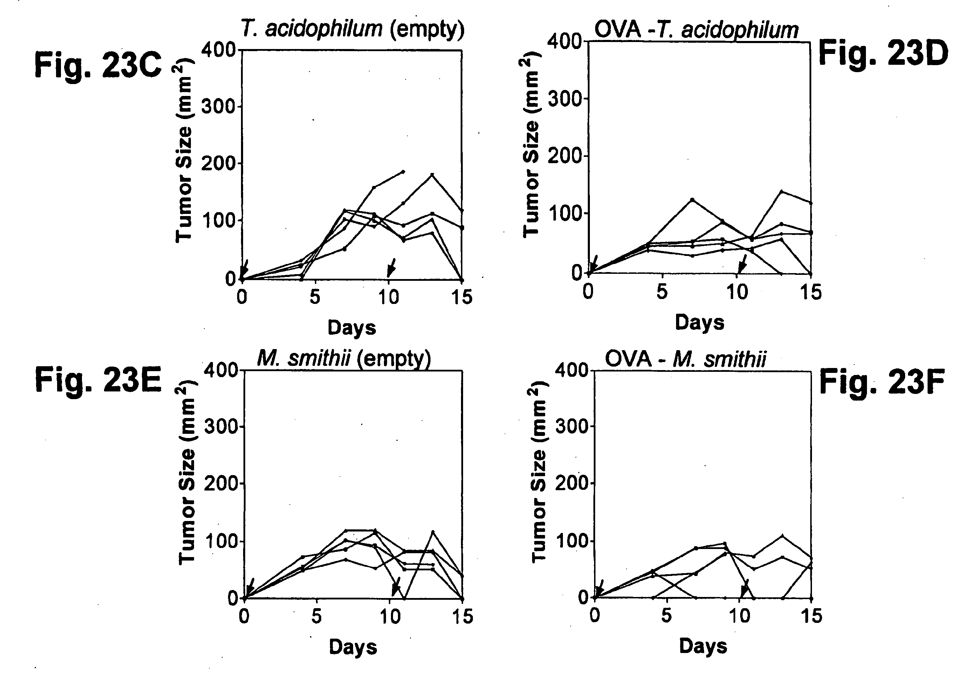

**Fig. 23** shows that immunization with either empty archaeosomes or with antigen encapsulated in archaeosomes results in regression of solid tumors. C57BL/6 mice were injected with 10 million EG.7 tumor cells. Naïve mice were not immunized (Fig. 23A). Other groups of mice were immunized on days 0 and 10 as indicated by arrows, with 15 µg OVA either in PBS (Fig. 23B), or encapsulated in *T*. *acidophilum* (Fig. 23D) or *M. smithii* (Fig. 23F) archaeosomes, or with empty *T*. *acidophilum* (Fig. 23C) or *M. smithii* archaeosomes (Fig. 23E).

### DETAILED DESCRIPTION OF THE INVENTION

The current invention shows that archaeosomes unexpectedly induce a strong and sustained antigen-specific CTL response to appropriate acellular protein or peptide antigen that is encapsulated in and/or associated with the archaeosome, without the need for inclusion of other immune modulating adjuvant in the vaccine formulation. This is clearly contrary to the prior art using conventional liposomes. Further, the ability of archaeosomes to simultaneously elicit MHC-I (CTL) and MHC-II (Th1 and Th2) responses, as well as appropriate strong and sustained memory responses, was also unexpected, and contrary to literature expectations of such vehicular delivery systems. Examples of the efficacy and utility of archaeosomes as immunomodulating carriers for appropriate acellular antigens is demonstrated herein in murine models, as protective immunity against infection by intracellular pathogens (*Listeria monocytogenes* and *Francisella tularensis*) and against cancer, both examples of diseases known to require induction of a strong CTL response for protection.

The skilled artisan will recognize that the antigen is selected based on the type of disease affecting the patient. The pathogen from which the acellular antigen is derived, or is based upon, could be from a bacterium, a virus, or a protozoan parasite, as examples. Such antigen could be the killed pathogen, or component extracted from the pathogen, such as a toxin or capsular polysaccharide produced by the pathogen, membrane protein, coat protein, cytoplasmic protein, protein fragments, peptide or other component. The antigenic peptide sequence could be chemically synthesized, for example from amino acids and/or polymerization, or produced by recombinant technology, both well known in prior art.

In another embodiment, the antigen is from a pathogen where cellular immunity is important to protect the immunized host against infection or re-infection. It will be well recognized by the skilled artisan that intracellular pathogens are one group of infectious agents that require the vaccinated host to mount cellular immunity to protect the host from the infectious challenge. Examples of intracellular pathogens (given for illustrative purposes only and not to limit the scope of the invention) are bacteria that cause tuberculosis *(Mycobacterium tuberculosis),* listeriosis *(Listeria monocytogenes),* tularemia *(Francisella tularensis),* and leprosy (*Mycobacterium leprae*)], viruses [members of the families adenovirus, coronavirus, herpes virus, orthomyxovirus, papovirus, paramyxovirus, picornavirus (the most readily acknowledged viruses being HIV which cause AIDS and the viruses that cause influenza)], and parasites that cause malaria *(Plasmodium* species), toxoplasmosis *(Toxoplasmosis gondii),* and leishmaniasis *(Leishmania* species).

In yet another embodiment, the antigen is a tumor associated antigen (i.e., antigen associated with neoplastic disease) which can be useful for prophylactic and/or therapeutic treatment of cancer in the immunized host. Tumor antigens are well known in the art. Illustrative examples are prostrate specific antigen, carcinoembryonic antigen, mucins and various melanoma antigens. The immunomodulating archaeosome carrier of the present invention is capable of activating antigen presenting cells (APCs) as well as presenting the antigen in the context of MHC class I and/or class II. Skilled artisans will appreciate that the type of immune reaction elicited will also depend on whether the antigen has the respective epitope(s) required for generating the class I/II immune reactions. Such appropriate antigens can be prepared readily by those skilled in the art, using traditional or modem synthetic or recombinant techniques.

In the present invention, it is required that the archaeosome be a carrier for the appropriate acellular antigen. One skilled in the art will recognize that this can be accomplished by encapsulating the antigen within the archaeosome and/or associating it with the archaeosome lipid layer using methods known in prior art for liposomes and other particulate delivery vehicles. This may be accomplished, for example, by either covalent or noncovalent (e.g., hydrophobic, adsorption) interaction between the liposome and the antigen. It will also be appreciated by the skilled artisan that the attachment of the peptide or protein antigen to the archaeosome can be quantitatively facilitated by linking a hydrophobic anchor, such as a fatty acyl group or a hydrophobic sequence of amino acids, to a terminal end of the peptide. However, for the current invention to work, such modification of the antigen is not a prerequisite.

The vaccine composition of the invention comprises the archaeosome as an immunomodulating carrier for the acellular antigen, and may include other pharmaceutically acceptable excipients (e.g., water, saline, glycerol, dextrose, pH buffering agents, bacteriostatic compounds and combinations thereof) that are compatible with the active components in the vaccine formulation, and not deleterious to the recipient thereof.

As can be appreciated by one skilled in the art, the compositions of the invention can be used to immunize a host in need of protection from infection by a specific infectious agent or at risk from developing a specific disease or tumor-associated ailment. The vaccine formulations of the invention will include an immune stimulating amount of appropriately selected acellular antigen. Immunization of the host can be accomplished by the normally acceptable routes of vaccine administration including parenteral routes such as subcutaneous (s.c.), intramuscular (i.m.), and intradermal (i.d.), and others (oral, intranasal, topical). The dosage of the vaccine formulation is administered in a manner compatible with the host to be immunized, the route for immunization, and in a manner that will be therapeutically effective, immunogenic and protective. The skilled artisan will easily be able to factor in-the various circumstances and determine the most appropriate regimen for immunization.

It is shown that as immunomodulating carriers, archaeosomes are superior to conventional liposomes and alum in eliciting an antigen-specific cytotoxic T cell response. This response is mounted early after vaccination, has a memory response lasting a prolonged period of time, and protects against challenge by the appropriate intracellular pathogen or cancer. The archaeosomes of the current invention are safe, non-toxic and cause no adverse reactions in the vaccinated host.

In the present invention, archaeosomes are defined as liposomes that are prepared from polar lipids extracted from one or more members of *Archaea,* or lipid(s) that mimic polar lipid structures found in members of *Archaea,* or from one or more polar lipid(s) purified in a biologically pure form from *Archaea.* The skilled artisan will appreciate that lipids that structurally mimic (such as those made by chemical synthesis) the polar lipids found in the *Archaea* could be used to make archaeosomes for the purposes of the current invention. The *Archaea* produce many different polar lipid structures that are useful to produce archaeosomes. Of the available species of *Archaea* as a class of organisms, we have selected several as illustrative examples as sources of lipids for preparing archaeosomes for the current invention.

The above disclosure generally describes the current invention. This invention will be better understood from the data given under the heading **"RESULTS AND DISCUSSION".** The data therein are for illustrative purposes only and are not intended to limit the scope of the invention. Changes in form and substitution of equivalents are contemplated as the circumstances may suggest appropriate.

### Definitions of some of the various terms used in this description:

The specific terms (definitions) used in the disclosure are intended in a descriptive sense and are not intended for the purposes of limitations. Antigen, an immunogen (e.g., derived from proteins, peptides, carbohydrates, lipids and mixtures thereof) to which an animal such as a human mounts an immune response; acellular antigen, an antigen that is non-replicating (not able to grow and multiply) and that may represent an extract from a pathogen/diseased tissue or a highly purified component thereof, additionally including such components that are chemically synthesized or produced by recombinant methods so as to mimic those found in the pathogen/diseased tissue; adjuvant, a substance or material with immune modulating (immunomodulating) and/or carrier activity which when administered with an immunogen enhances the immune reaction to that immunogen; host, any animal, including a mammal, e.g., a human, cow, pig, horse, cat, dog; naïve T. cell, a T cell that has not been exposed to a foreign antigen or to a cryptic autologous antigen; activated T cell, a T cell that is undergoing mitosis and/or cell division, one that is in the G₁ phase, G₂ phase, S phase or the M phase of the cell cycle; costimulatory or accessory molecule, a molecule that promotes the antigen-MHC interaction with the T cell receptor, these molecules such as (illustrative examples only) B7.1 (bind to CD28) and B7.2 (bind to CD28) facilitate various functions such as initial binding, stabilizing the association, signal transduction, separation etc.; memory response, immune cells that have previously been exposed to an antigen, are in resting stage but capable of being activated upon exposure to the antigen again; memory T cell, T cell that is capable of mounting a memory response; conventional phospholipid or ester phospholipid, a glycerolipid in which the hydrocarbon chains are linked to the glycerol backbone via ester bonds; ether lipid, a glycerolipid in which the hydrocarbon chains are linked to the glycerol backbone via ether bonds; archaeal or archaeobacterial polar lipid, polar lipid derived from a member of the class *Archaea* (synonymous to *Archaeobacteria)* or a lipid that is chemically synthesized to mimic the unique structure of archaeal polar lipid(s); liposome, closed vesicle made of lipid membranes which entraps an aqueous volume, the liposome may be unilamellar, oligolamellar or multilamellar; conventional liposome, a liposome prepared from conventional phospholipid(s) and in some cases including a sterol and may include other compounds that are entrapped within the vesicle or associated with the lipid membrane; archaeosome, a liposome prepared from one or more of the polar lipids that are unique to the species in the class *Archaea,* including those vesicles made from archaeobacterial polar lipid(s) or lipid(s) that structurally mimic the polar lipids found uniquely in archaeobacteria, similar to conventional liposomes archaeosomes may include other compounds that are entrapped within the vesicle or associated with the lipid membrane layer; vesicle, liposome or archaeosome. The name of the archaeobacterium associated with the word archaeosome (for example, *T. acidophilum* archaeosome, or archaeosome of/from *T. acidophilum*) indicates that the archaeosome is made with lipids extracted from that specific archaeobacterium, and unless stated to the contrary, from the total polar lipids (TPL) extracted from that archaeobacterium.

### MATERIALS AND METHODS

### Growth of archaea and extraction of lipids

*Halobacterium salinarum* ("*H. cutirubrum*") (ATCC 33170), *Methanobrevibacter smithii* ALI (DSM 2375), *Methanosphaera stadtmanae* MCB-3 (DSM 3091), *Thermoplasma acidophilum* 122-1 B3 (ATCC 27658), and *Natronobacterium magadii* (ATCC 43099) were cultivated as described earlier (Choquet et al., 1994). Total lipids were extracted from frozen cell pastes, and the total polar lipids (TPL) were collected as the acetone-insoluble fraction (Choquet et al., 1994). When needed, polar lipids were isolated in a biologically pure form by thin-layer chromatography from either TPL or total lipid extracts (Kates et al., 1993).

### Antigens

Antigens tested included fatty acid free-bovine serum albumin (BSA), hen egg lysozyme (HEL), and ovalbumin (OVA), all purchased from Sigma Chemical Co.

To test for the ability of archaeosomes to induce protection in mice against infection by *Listeria monocytogenes,* a synthetic peptide antigen spanning amino acids 90 to106 of listeriolysin was entrapped in vesicles. The known H-2K^{d} MHC-Class-I-restricted-immunodominant nonamer epitope (GYKDGNEYI) of listeriolysin (Pamer et al., 1991) was synthesized either as the free nonapeptide, or as a dipalmitoylated 13-mer peptide (PAM₂-KSSKGYKDGNEYI-OH), or as a dipalmitoylated 20-mer peptide (PAM₂-KSSKGYKDGNEYIWEKKKK-OH) containing extensions upstream, or upstream and downstream of the nonamer epitope, respectively. Peptides were synthesized using a MilliGen® 9050 continuous flow peptide synthesizer and FMOC chemistry as previously described (Barbier et al., 1997), but width NovaSyn® TGT resin (Novabiochem) as the support. Peptide purification and analytical HPLC were performed with diphenyl silica columns (Vydac®) using an acetonitrile gradient in 0.1 % trifluoroacetic acid/water. Purity was estimated to be >97% by analytical HPLC. Product identities were confirmed by matrix-assisted laser desorption (MALDI) mass spectrometry. Masses determined / (expected) were: free 9-mer, 1058.69 (1058.12), dipalmitoylated 13-mer, 1965.27 (1965.41), and dipalmitoylated 20-mer, 2806.42 (2805.51). The dipalmitoylated peptides [(PAM)₂ peptides] are referred to also as 13-mer or 20-mer lipopeptides.

Outer membranes of *Francisella tularensis* were isolated via an N-lauroylsarcosine method (Leslie et al., 1993). Bacterial paste was frozen and thawed once, followed by suspension in 10 mM Tris-HCl, pH 8.0, and broken by French pressure cell treatment. Following a low speed centrifugation to remove unbroken cells, 0.55% N-lauroylsarcosine (w/v) was dissolved in the supernatant and incubation continued for 30 min. Outer membranes were harvested and washed by centrifuging at 42,000 x g for 1 h.

### Preparation and characterization of archaeosomes and conventional liposomes

Archaeosomes were prepared from the total polar lipids (TPL) extracted from the indicated archaeobacteria above, except for PGP-0-CH₃ and PG archaeosomes. The former were prepared from the purified archaetidyl glycerolphosphate-0-methyl (PGP-0-CH₃) polar lipid (Kates et al., 1993) isolated from *H. salinarum* with a purity of at least 79% (the remainder being PG), and the latter from archaetidyl glycerol (PG) isolated from *N*. *magadii* with a purity of >98% as determined by negative-ion fast atom bombardment mass spectrometry. L-α-dimyristoylphosphatidylcholine (DMPC), L-α-dimyristoylphosphatidylglycerol (DMPG) and cholesterol (CHOL) were purchased from Sigma Chemical Co., St. Louis, MO, for the preparation of conventional liposomes (Con. Liposome), defined herein as DMPC:DMPG:CHOL or PC:PG:CHOL (1.8:0.2:1.5 molar ratio).

For encapsulation (entrapment) of BSA, OVA, or HEL, the vesicles were prepared by pressure extrusion through 400 nm filters at ambient temperature, using Liposofast® apparatus (Avestin Inc., Ottawa, Canada). Pyrogen-free, sterile, deionized distilled water and glassware were used throughout, for all vesicle preparations. Briefly, 20 mg of dried lipid was hydrated over a period up to 16 h at 35°C, in 1-ml phosphate-buffered saline (PBS, 10 mM potassium phosphate buffer at pH 7.14, plus 160 mM NaCl) containing the protein antigen (10 mg/ml) to yield multilamellar vesicles. The multilamellar vesicles were pressure extruded as described above to obtain predominantly unilamellar vesicles. Antigen that was not associated with the pressure extruded vesicles was removed by ultracentrifuging (200,000 x gₘₐₓ for 30 min) and washing, thrice, from 7 ml volumes of PBS. All vesicle preparations were characterized by measuring salt-free dry weight, and the mean vesicle diameters were determined by number-weighted Gaussian size distributions using a Nicomp® Particle sizer (model 370, Nicomp, Santa Barbara, CA). The amount of respective protein associated with the vesicles (encapsulated within vesicle and vesicle surface exposed/adsorbed) was estimated, after lipid removal (Wessel and Flugge, 1984), by SDS Lowry and comparison with standard curves constructed for the relevant protein. Similar immunological properties were found for archaeosomes prepared by either pressure extrusion or dehydration-rehydration, described below.

To encapsulate the (PAM)₂ peptides or the outer membranes of *F*. *tularensis* in vesicles, a dehydration-rehydration method was used. Briefly, 30 mg of the total polar lipids of the indicated archaeobacterium, or the mixture of lipids for the conventional liposomes, were dried under nitrogen and *in vacuo* for about 1 h, followed by addition of 2.5 ml water, followed by addition of the antigen (0.5 - 3 mg) dissolved in 0.15 ml dimethylsulfoxide (DMSO) in the case of the (PAM)₂ peptides (lipopeptides), or water in the case of watersoluble antigens, or outer membranes. Hydration was allowed to proceed for 1 h at 35°C in a shaker water bath, followed by vesicle size reduction in a water bath sonicator to achieve predominantly unilamellar vesicles with an average diameter of less than 100 nm. The preparations were then lyophilized and rehydrated slowly in 0.3 ml water and incubated at 35°C in a shaker for 1 h. Following addition of 1.2 ml PBS (10mM phosphate buffer, pH 7.1, 160 mM NaCl), the preparation was filtered through a 0.45 µm sterilizing filter (Millipore Millex syringe filter units). The amount of lipopeptide antigen entrapped in the various vesicle types was estimated by SDS-Lowry assay using the corresponding unentrapped antigen as the standard, and equivalent amounts of antigen-free vesicles as the blank control. Because essentially all of the palmitoylated peptides were incorporated into archaeosomes by the method used, a separation step to remove unbound antigen was unnecessary. However, in the case of other antigens, unentrapped antigen was removed by centrifuging and washing the liposome pellet with PBS. In most cases, vesicles containing antigen averaged 150 ± 100 nm in diameter.

The dipalmitoylated 20-mer peptide was prepared as micelles for vaccinations to test the effect of antigen without entrapment in liposome. The 20-mer lipopeptide dissolved in DMSO was mixed (by vortexing for 2 min followed by brief sonication in a bath sonicator) with PBS containing 0.01 % trifluoroacetic acid to achieve a final concentration of 0.4 mg / ml antigen and 10% v/v DMSO. The antigen was further diluted in PBS to the desired final concentration.

### Cell lines

J774A.1 and IC21 (macrophages) cell lines were obtained from ATCC and maintained in RPMI 1640 medium (Life Technologies, Grand Island, NY) supplemented with 8% FBS (Hyclone, Logan, UT) and 10 µg/ml gentamycin (Life Technologies). EG.7, a subclone of EL-4 stably transfected with the gene encoding OVA (Moore et al., 1988), was also obtained from ATCC but maintained in RPMI plus 8 % FBS, additionally containing 400 µg /ml G418 (Rose Scientific Ltd., Edmonton, Alberta, Canada). B16-OVA cells (a melanoma cell line expressing OVA peptides) were a gift from Dr. Edith M. Lord (University of Rochester, Rochester, NY), and were maintained in RPMI 1640 medium supplemented with 8% FBS. The Phem 3.3 cell line (MHC class II negative cell line) was a gift from Dr. M. J. Bevan (University of Washington, Seattle, WA). Phem 3.3 is a subclone of the mastocytoma cell line P815 that has been stably transfected with the gene encoding listeriolysin (Pamer et al., 1991). These cells were routinely maintained in RPMI containing 8 % FBS, and 400 µg/ml G418 (Rose Scientific Ltd., Edmonton, Alberta, Canada). The parent cell line, P815, was obtained from the ATCC and maintained in RPMI supplemented with 8 % FBS. The Wehi 164-13 cell line used for TNF bioassay was obtained from Dr. T. R. Mosmann (University of Rochester, Rochester, NY) and maintained in RPMI containing 8% FBS. All cells were cultured at 37°C, under 8% CO₂ in a humidified atmosphere.

### Generation of bone-marrow derived dendritic cells (DCs)

Bone marrow was flushed from the femurs and tibias of one to three normal BALB/c mice, and single cell suspensions made by pressing them through Falcon® 2360 cell strainers (Becton Dickinson, Franklin Lakes, NJ) using a sterile 1-ml syringe plunger. Cells were counted and resuspended at 1x10⁶ cells/ml in RPMI medium supplemented with 8 % FBS and 5 ng/ml of recombinant murine GM-CSF (ID Labs, London, ON, Canada) in a Falcon® tissue culture flask (Becton Dickinson) and cultured for 6-8 days at 37°C in 8% CO₂, Non-adherent cells were removed at days 2 and 4 of culture, and fresh RPMI plus 8% FBS containing GM-CSF (granulocyte-macrophage colony stimulating factor) was added. On the day of the experiment, non-adherent cells were harvested, washed, counted and used. The dendritic cell preparations were consistently >80 % CD11c⁺ by flow cytometry.

### Mice

Specific-pathogen-free inbred, 6-8 week old female BALB/c and C57BU6 mice were purchased from Charles Rivers Laboratories (Montreal, Que.). CS7BL/6J CD4⁺ T cell deficient mice and their controls were obtained from The Jackson Laboratory (Bar Harbor, Maine). They entered experiments when they were 8-12 weeks old. Mice were maintained and used in accordance with the recommendations of the current edition (1993) of the Canadian Council on Animal Care Guide to the Care and Use of Experimental Animals.

### Immunization

Groups of 3-6 mice received immunizations of the antigen in PBS (no adjuvant), in alum or Freunds adjuvant (FA), or entrapped in archaeosomes or conventional liposomes, as specified in the figure legends or table footnotes. Immunization volume was 100-200 µl, antigen dose was 8-30 µg/injection, and vesicle lipid concentration 0.5-1.5 mg/injection. For alum immunizations, the antigen was adsorbed onto Imject® Alum (Pierce, Rockford, Illinois) according to the manufacturer's protocol. Immunization routes were intraperitoneal (i.p.), or subcutaneous (s.c.) injected at the base of the tail.

### Infection and tumor challenges In murine models

All vaccines tested in the infectious pathogen challenge using appropriate murine models were administered subcutaneously, at the base of the tail, in 0.1 ml volume in PBS. Various vaccination regimens were employed as stated in the relevant sections of the results. For challenge in the listeriosis model, at various stated times post-vaccination, control and vaccinated mice were challenged with an intravenous inoculum of either *Listeria monocytogenes* strain 10403S prepared as described previously (Conlan, 1997), or with *Salmonella typhimurium* strain C5R. At various stated times post challenge, mice were killed by CO₂ asphyxiation, and their livers, spleens and other organs removed, as required. These organs were homogenized and plated on brain heart infusion agar containing 50 µg/ml streptomycin. Bacterial colonies were counted (colony forming units, CFU) after incubating the plates for 24 h at 37 °C. Bacterial burdens (CFU/organ) in the tissues of various test groups were statistically analysed using the Mann Whitney rank sum test, and were considered to be significantly different from one another at p<0.05.

In the case of *Francisella tularensis* infection model, the challenge to control and immunized mice was delivered as an aerosol. Small particle aerosols in the 4-6 µm range are generated in a Lovelace® nebulizer operating at 40 p.s.i. Once generated, aerosols are delivered to an exposure chamber. For exposure, mice are housed in restraining cones from which only their external nares protruded into the aerosol stream. 3 days after the pathogen challenge (unless stated otherwise) the mice were killed, and the liver, spleen and lungs were removed as described above: The organs were homogenized and plated on cysteine heart agar supplemented with 1% (w/w) hemoglobin. Bacterial colonies were counted after incubating the plates for 24 h at 37°C. Bacterial burdens (CFU/organ) In the tissues of various test groups were statistically analysed using the Mann Whitney rank sum test, and were considered to be significantly different from one another at p<0.05. For both models of infection above, the lower limit of pathogen detection in the liver and spleen was 100 CPU/organ and 50 CFU for lungs.

For the EG.7 tumor model, C57BL/6 mice (5 per group) were Immunized s.c. at the base of the tail with the vaccines described. Mice were injected (s.c.) with 10X10⁶ EG.7 tumor cells in the mid-back region, to initiate the tumor challenge. The tumor model was used to evaluate the efficacy of archaeosomes for prophylactic (mice pre-immunized before tumor challenge) and therapeutic (tumor cells implanted and mice immunized at same time) applications. Mice were monitored regularly for tumor progression and once the tumor reached a palpable size, the length (L) and width (W) were measured with digital calipers and tumor growth recorded as the product of L X W (mm²). Mice were euthanized when tumors reached a maximum width of 17 mm.

For the metastatic melanoma model, C57BL/8 mice were immunized (s.c.) on days 0 and 21 with 25 µg OVA encapsulated in *M*. *smithii* archaeosomes. Eight weeks after the first immunization, groups of naïve control mice and the immunized mice were injected (i.v.) with B16-OVA cells (5 X 10⁵/mouse) to initiate tumor challenge. Mice were euthanized 14 days post-challenge, and the black metastatic foci in the lungs were enumerated by visual counting.

### Antigen-specific proliferation of splenocytes

Splenocytes were obtained on day 28 from individual mice immunized on days 0 and 21. Selective lysis of RBCs was performed using Tris-buffered ammonium chloride, pH 7.2 (Sigma Chemical Co.). Cells were washed twice, and triplicate cultures set up (72 h, 7 % CO₂) in RPMI 1640 medium (Gibco-BRL, Life Technologies Inc., Grand Island, NY) in 96-well round bottom microtitre plates (Falcon^{®}. Becton and Dickinson) for proliferation in the presence or absence of antigen. The serum supplement used for culture included either Defined equine serum (2 %, for BSA-stimulated cultures) or Fetal bovine serum (8 %, for HEL- and OVA-stimulated cultures), obtained from Hyclone Laboratories Inc., Logan, Utah. Wells were pulsed with 1 µCl [³H]thymidine. (ICN Pharmaceuticals Canada, Montreal, Quebec) for 18 h, harvested onto glass fibre filters, and the radioactivity incorporated into the cells (measure of proliferative response) determined by liquid scintillation counting. The proliferative response was expressed as counts per minute (CPM) or as CPM X 1000 (kCPM) ± SEM of mice in each group.

To assess listerlolysin-specific stimulation, spleen cells (5 x **10⁵)** from Immunized mice were cultured In triplicate, in 96-well microtitre plates (Falcon^{®}, Becton Dickinson, Franklin Lakes, NJ), with various concentrations of the free nonamer peptide. After 72 h, supernatants were collected and assayed for IFN-γ production by a sandwich ELISA.

### Cytokine assays

Cytokines secreted into the supernatants of antigen-stimulated cultures were measured by a sandwich ELISA method (Mosmann and Fong, 1989). Antibody pairs used included RA-6A2 (ATCC HB170), and XMG1.2-biotin (Cherwinski et al., 1987) for IFN-γ; and 11B11 (Ohara and Paul, 1985) and BVD6-24G2-biotin (Pharmingen Canada Inc, Mississauga, Canada) for IL-4. IFN-γ and IL-4 standards were purchased from ID Labs, London, Canada. Duplicate standard curves were included on each plate. The sensitivity of the ELISAs were as follows; IFN-γ >100 pg/ml and IL-4 >15 pg/ml. TNF secreted into the supernatants of archaeosome-activated APC cultures was measured by a bioassay on Wehi 164-13 cells (Sad et al.. 1995). The sensitivity of the TNF assay was 0.1 pg/ml.

### Cell cycle analysis

Cell cycle analysis was performed by quantitation of the incorporation of DNA-binding dye, propidium iodide by Flow cytometry. Briefly, splenocytes (10⁶) were stained with FITC-conjugated anti-CD4 or anti-CD8 antibodies (Pharmingen Canada Inc.) for 30 min on ice in 50 µl of RPMI medium containing 8 % fetal bovine serum (FBS). Cells were then washed and fixed overnight at 4°C in 1 ml 70 % ice-cold ethanol. Fixed cells were stained with propidium iodide (Calbiochem, La Jolla, CA) in the presence of ribonuclease A (100 U/ml, Boehringer Mannheim, Laval, Canada) and analyzed by Flow Cytometry (EPICS^{®} XL, Beckman Coulter Corp., Hialeah, FL). DNA content profiles gated on CD4⁺ and CD8⁺ T cells were obtained using the EXPO^{®} software (Beckman Coulter Corp.). As the ploidy of the DNA increases as cells enter synthetic and mitotic phases, each cell incorporates greater amounts of propidium iodide. Thus, the percentages of cells in various phases of the cell cycle [apoptotic, G1 (resting), S (synthetic), and G2/M (mitotic)] were calculated based on DNA content.

### Evaluation of antibody titres

Mice were bled either from the veins at the end of the tail or by cardiac puncture, and blood collected in Microtainer^{®} serum separator tubes (Becton Dickinson, Franklin Lakes, NJ). After allowing the blood to clot (1 h at 4°C), the serum was separated by centrifugation and frozen at -20°C until assayed. The antibody levels were determined by indirect antigen-specific ELISA. Briefly, ELISA plates (EIA microtitration plates, 96-well flat bottom, ICN Biomedicals Inc., Aurora, OH) were coated with antigen in PBS (10 µg/ml), and serial two-fold dilutions of serum (from individual mice) were assayed in duplicate. Horseradish peroxidase-conjugated goat anti-mouse immunoglobulin (IgG + IgM) revealing antibody (Caltag, San Francisco, CA) was used to determine total antibody titres of sera. The reactions were developed with ABTS microwell peroxidase system (Kirkegaard and Perry Laboratories, Gaithesburg, Maryland) and absorbance determined at 415 nm after 15 min at room temperature. Antibody titres are represented as endpoint dilutions exhibiting an optical density of 0.3 units above background. Samples pertaining to each experiment were evaluated in the same assay.

### CTL assays

Effector cells were prepared from the splenocytes from immunized and naïve mice. 30 x 10⁶ spleen cells were co-cultured with 5 x 10⁵ irradiated EG.7 cells, in 25 cm² tissue culture flasks in an upright position. The culture medium was 10 ml of RPM1 1640 supplemented with 8 % FBS and 0.1 ng/ml IL-2. After 5 days at 37 °C and 8 % CO₂, the cells were recovered, washed, counted and used as effectors for the CTL assay. Target cells were EG.7 or EL-4 cells. EL-4, unlike EG.7, do not present a MHC-1 OVA epitope and thus served as negative controls for non-specific killing. Target cells (10⁷) were labeled with 100 µCi ⁵¹Cr in 50 µl of RPM1 plus 8% FBS medium for 45 min. Targets were washed and various ratios of effectors and targets were co-cultured for 4 h in 96-well, round bottom tissue culture plates. The culture supernatants were collected and radioactivity measured by gamma counting. Percent specific lysis was calculated as 100 x [(cpm experimental - cpm spontaneous)/cpm total - cpm spontaneous)]. One lytic unit is defined as the number of effector cells per 10⁶ spleen cells that yield 20% specific lysis of a population of 2.5 x 10⁴ target cells.

To determine CTL activity in spleens of mice immunized with the listeriolysin lipopeptide, 30 x 10⁶ spleen cells were co-cultured with 5 x 10⁵ irradiated (10,000 rads) Phem3.3 cells in 10 ml of RPMI plus 8 % FBS containing 0.1 ng/ml IL-2, in upright 25 cm² tissue culture flasks (Falcon^{®}). After 5 days (37°C, 8% CO₂), the cells were recovered from the flask and used as effectors in the standard ⁵¹Cr-release CTL assay, as detailed above. Targets were either Phem 3.3 cells (specific target expressing listeriolysin peptide) or P815 (non-specific control) cells.

### In vitro activation of antigen presenting cells (APCs)

J774A.1 or bone marrow derived dendritic cells were incubated with 25 µg/ml of empty archaeosomes or conventional liposomes, *in vitro* in 24-well tissue culture plates (Falcon^{®}) at appropriate cell densities as stated. Alternatively, cells were activated with 10 µg/ml LPS (obtained from *E. coli*, a gift from our colleague Dr. M. B. Perry). After 24 h, at 37°C, 8 % CO₂, in a humidified atmosphere, the cells were recovered and stained for various cell surface markers or used as APCs (after irradiation at 2500 rads) in T cell proliferation assays.

### Archaeosome injections, and peritoneal exudate preparations

BALB/c or C3H/HeJ mice were injected intraperitoneally with empty archaeosomes (1mg lipid/injection). At various time points after injection, mice were euthanized, and peritoneal exudate cells were recovered by performing peritoneal lavage with 10 ml of warm RPMI plus 8% FBS medium. Erythrocytes were then selectively lysed with Tris-buffered ammonium chloride, pH 7.2 (Sigma Chemical Co.). The cells were washed, resuspended in RPMI plus 8% FBS, counted and analyzed for cell surface marker expression, used for cell sorting, or used as APCs (after irradiation) for T cell allo-stimulation assays.

### T cell allo-stimulation assay

C57BL/6 (H-2K^{b}) splenocytes obtained from naïve mice were enriched for CD4⁺ or CD8⁺ T cells by passing through appropriate T cell columns (Cytovax, Edmonton, Canada) according to the manufacturer's instructions. Briefly, splenocytes (100 X 10⁶) were incubated with a rat anti-mouse CD4 or CD8 antibody, loaded onto glass bead columns coated with goat anti-rat Ig and sheep anti-mouse Ig, and eluted. Cells after passing through the column were 80-90 % pure for the appropriate T cell as determined by Flow cytometric analysis. T cells thus obtained, were cultured with irradiated (2500 rads) allo-APC (H-2K^{b}) at various APC:T cell concentrations, in triplicate, in RPMI plus 8% FBS medium, in 96-well microtitre plates (Falcon^{®}). After 72 h, an aliquot of the culture supernatant was collected for cytokine analysis. To determine T cell proliferation, the cultures were pulsed with 1 µCi [³H] thymidine (ICN Pharmaceuticals Canada, Montreal, Canada) for 18 h, harvested onto glass-fiber filters and the radioactivity incorporated determined by liquid scintillation counting.

### Flow cytometric analysis and sorting

For flow cytometric analysis, cells were incubated on ice (10⁶ cells in 50 µl of RPMI plus 1% FBS) with anti-mouse CD32/CD16 (FcγII/III receptor). After 30 min, aliquots were washed and incubated in 50 µl of RPMI plus 1 % FBS with the different FITC, PE or biotinylated anti-mouse antibodies as stated in the figure legends. The antibodies used for the various experiments included; Mac 1α (CD11b), F4/80, CD11c, CD80 (B7.1), CD86 (B7.2), H-2K^{d} (MHC class I), Ia^{d} (MHC class II), B220, CD4, CD8, CD28, CD44 and LFA-1. All antibodies were obtained from PharMingen Canada Inc (Mississauga, Canada) except for F4/80 that was obtained from Cedarlane laboratories (Homby, Ontario, Canada). Antibody incubation was for 30 min on ice. Cells stained with biotinylated antibodies were subsequently incubated with streptavidin-FITC after thorough washing. Cells were fixed in 1% formaldehyde in PBS and analyzed by flow cytometry (EPICS^{®} XL; Beckman Coulter Corp., Hialeah, Canada) using their EXPO^{®} software.

For flow cytometric cell sorting, peritoneal exudate cells (10 X 10⁶/ml) were stained with 5 µl of the anti-mouse CD32/CD16 antibody, followed by 5 µl of PE labeled, anti-mouse Mac 1α, or B220 antibody for 30 min on ice. Cells were then washed and resuspended in 1 ml of RPMI 1640 supplemented with 1% FBS. Cells were sorted on EPICS^{®} Elite ESP (Beckman Coulter Corp.), and collected in R8 medium. Subsequently an aliquot of the sorted cells was analyzed on EPICS^{®} XL, to confirm purity.

### RESULTS AND DISCUSSION

### Archaeosomes facilitate antigen-specific proliferation of spleen cells

The induction of a cell-mediated response against antigens is often critical for sustained and effective immunity, particularly against intracellular pathogens. One of the first indicators of an effective cell-mediated immune response mediated by CD4⁺ T cells is the ability of lymphocyte populations of immunized mice, to respond to soluble antigen (Ag) *in vitro.* Mice (BALB/c and/or C3H/HeJ) were immunized with BSA (Fig. 1A and 1B), HEL (Fig. 1C), or OVA (Fig. 1D) encapsulated in the indicated archaeosomes or in conventional liposomes (Con. Liposomes), or adsorbed onto alum (Alum). Antigen-alone (No adjuvant) controls were included as indicated. BSA, HEL and OVA were used at 10, 10 and 15 µg, per injection, respectively. Immunizations were i.p. or s.c. on days 0 and 21. Spleens were harvested on day 28 and antigen-specific proliferation of splenocytes assessed as described under Materials and Methods. Data represent the mean antigen-dependent proliferative response, expressed as counts per minute (CPM) x 1000 (kCPM) ± SEM of mice in each group [n = 5 (Fig. 1A, 1B), n = 6 (Fig. 1C) and n = 3 (Fig, 1D)].

A BSA-specific proliferation of spleen cells after i.p. immunization was observed (Fig. 1A). Dose-dependent proliferation to BSA was observed only in spleen cells of mice that had been immunized with BSA entrapped in archaeosomes (BSA-archaeosomes). Conventional liposomes as well as alum failed to induce significant proliferation. Furthermore, the proliferative response of spleen cells following archaeosome immunization, was seen in both BALB/c, and C3H/HeJ (LPS hyporesponsive) mice (Fig. 1 B). Similar antigen-specific proliferation was seen after immunization of mice with HEL-archaeosomes (Fig. 1C) and OVA-archaeosomes (Fig. 1D). Immunization with archaeosomes by the s.c. route resulted in similar responses, that were higher than those with conventional liposomes (Fig. 1D, lower panel).

Similar to the conventional liposomes, greater than 85% of the BSA associated with the archaeosomes was entrapped within the vesicles, with the balance being exposed on the vesicle surface (data not shown), as determined by BSA analyses of the respective vesicles before and after proteolysis treatment.

### Archaeosomes induce Th1 and Th2 cytokine production by antigen-stimulated spleen and lymph node cultures

Antigen-specific lymphocyte proliferation results in activation of specific T helper responses and cytokine production. While Th2 (IL-4) responses are important for aiding antibody production by B cells, Th1 (IFN-γ) responses are important for induction of effective cell-mediated Immunity. Th1 and Th2 responses may be dichotomous and antigens may themselves skew immunity towards one or the other (Krishnan and Mosmann, 1998). A superior adjuvant on the other hand should possess the desirable capacity of inducing both Th1 and Th2 responses irrespective of antigenic nature.

BALB/c mice were immunized (days 0 and 21) i.p. with 10 µg HEL (Fig. 2A), or s.c. with 15 µg OVA (Fig. 2B), encapsulated in *M. smithii or M. stadtmanae* archaeosomes. Groups of mice immunized with HEL alone controls (i.e., antigen alone, No adjuvant). HEL encapsulated in conventional liposomes (Con. Liposome), and HEL admixed with alum (Alum) were included as shown in the figure. Spleens were harvested on day 28, and cells from each mouse were stimulated in triplicate with varying doses of the appropriate antigen *in vitro* for 72 h. Cytokines produced in 72 h supernatants were determined by ELISA. Values represent mean ± SEM of cytokine production by spleen cells of mice in each group [n = 6 (Fig. 2A), n = 3 (Fig. 2B)]. Substantial IFN-γ production was seen only in cultures of cells obtained from mice immunized with HEL-M. *smithii* archaeosomes (Fig. 2A). The IFN-γ levels showed a clear dose dependent production in response to stimulation by the antigen. Alum failed to evoke any IFN-γ production. On the other hand, both archaeosomes and alum induced IL-4 production. Conventional liposomes induced neither IFN-γ nor IL-4. The ability of *M. smithii* archaeosomes to induce Th1 (IFN-γ) as well as Th2 (IL-4) responses to entrapped antigens was further corroborated using another antigen system. Immunization of mice with OVA-archaeosomes by the s.c. route also resulted in production of both IFN-γ and IL-4 by spleen cells (Fig. 2B). After s.c. immunization with archaeosomes, cytokine production was detected even in popliteal lymph node cultures (data not shown). Even the potency of s.c. immunization failed to evoke substantial IFN-γ production by alum. Once again, conventional liposomes induced neither IL-4 nor IFN-γ.

Antigen-dependent IFN-γ production by cultured spleen cells (assayed as described in Fig. 2) obtained (on day 28) from BALB/c mice immunized s.c. (days 0, 21) with 15 µg of OVA encapsulated in *T*. *acidophilum* or *H*. *salinarum* or *M*. *smithii* archaeosomes was also observed (Fig. 3). Moreover, the IFN-γ produced (values represent means ± SEM of cytokine production by spleen cells of mice in each group, n=3) by spleens from OVA-archaeosome immunized mice was considerably higher than that seen in mice immunized with equivalent amount of OVA encapsulated in conventional liposomes (Con. Liposomes).

### Induction of T-cell memory by archaeosomes: increased cell cycling of CD4⁺ cells

Female BALB/c mice that were immunized (i.p.) on days 0 and 14 with BSA (15 µg/injection) encapsulated in *T*. *acidophilum* archaeosomes (0.53 mg/injection), or admixed with alum, or In PBS alone (No adjuvant) were boosted with 25 µg of BSA in PBS at days 210 and 334. Ag-archaeosome immunization induced a moderately enhanced primary antibody response that was sustained for nearly 200 days (P < 0.02 by ANOVA, for all time points compared to no-adjuvant immunization). Following an antigen alone boost on day 210, a remarkable memory antibody response was seen (approximately 2 log enhancement, P = 0.0004) that was sustained to at least day 300. On the other hand, alum, despite inducing an enhanced primary response that was comparable to that with archaeosomes, showed a subsequent drop in antibody titres and failed to evoke a significant recall response to antigen alone boosts (data not shown).

Seven days after the antigen alone boost at day 334, spleen cells were harvested, red blood cells were selectively lysed, and cells within each group were pooled for analysis of cell cycling. Cells were then stained with anti-CD4⁺ and anti-CD8⁺ FITC labeled antibodies, and cell cycling on gated CD4⁺ cells was analyzed by quantitating propidium iodide incorporation using Flow cytometry as described under Materials and Methods. 20,000 events per sample were acquired. Profiles in Figure 4A indicate the CD4⁺ (FL1) gate. Plots in Figure 4B indicate the percentage of CD4⁺ cells in various phases of the cell cycle [based on PI (FL3) content] in each group. As the amount of DNA per cell increases, the greater the incorporation of PI, and greater the number of cells in synthetic and/or mitotic phase. 'A' denotes apoptotic, 'G1' resting, 'S' synthetic, and 'G2/M' mitotic phase. The frequency of CD4⁺ staining in populations of spleen cells obtained from either BSA alone control (No adjuvant), BSA-alum (Alum), or BSA-*T*. *acidophilum* archaeosome (*T. acidophilum)* immunized mice is shown in Figure 4A. The total CD4⁺ cell number was increased slightly from 29 % in the controls to 37 % in the archaeosome-immunized group. Comparison, among the various groups, of cell cycling profiles of the CD4⁺ cells revealed that antigenic challenge of *T*. *acidophilum* archaeosome immunized mice resulted in a dramatic increase in S (synthetic) and G2/M (mitotic) phase cell numbers (Fig. 4B). This was accompanied by a concomitant decrease in cells in the G1 (resting) phase. In contrast, alum immunization evoked no change in the percentages of cells in the various phases when compared to the no-adjuvant control. No change was noted in the percentage of synthetic/mitotic phase CD8⁺ cell numbers between the different groups (data not shown), which is expected as soluble antigen challenge may not lead to MHC Class I presentation.

### Archaeosomes induce CTL responses to encapsulated OVA

C57BL/6 mice were immunized i.p., on days 0 and 21 with 15 µg of OVA in PBS (No adjuvant), or encapsulated either in conventional liposomes (Con. Liposomes) or in *M*. *smithii* archaeosomes *(M. smithii),* or adsorbed (admixed with) onto alum (Alum). Spleens were obtained on day 28, and pooled spleen (n=3/group) cells were stimulated with irradiated EG.7 cells for 5 days, and 4 h CTL activity against ⁵¹Cr labelled targets assessed as described under Materials and Methods. CTL data represent % specific lysis of triplicate cultures ± SD at various Effector:Target (E:T) ratios on EL-4 (non-specific target) and EG.7 (specific target expressing OVA peptides) cells (Fig. 5A). Spleen cells from OVA-archaeosome immunized mice exhibited strong CTL activity (% specific lysis) towards EG.7 but not the negative control EL-4 cells (Fig. 5A). A relatively low amount of antigen was used for these experiments (15 µg OVA in about 1.0 mg lipid), reiterating the potency of CTL response by OVA-archaeosomes. Spleen cells from the OVA-conventional liposomes and from the no adjuvant (OVA alone in PBS) immunized mice demonstrated no CTL activity, whereas the CTL activity of spleens from OVA-alum immunized mice was minimal.

The immunodominant CTL epitope in ovalbumin for H-2K^{b} haplotype, has been shown to be OVA₂₅₇₋₂₆₄ (SIINFEKL) (Rotzschke et al., 1991). To test whether the CTL response induced by archaeosomes correlated to presentation of this epitope, macrophage cells (IC21) were pulsed with the OVA₂₅₇₋₂₆₄ peptide for 2 h *in vitro,* and then used as stimulators for spleen cells obtained from the mice immunized in Figure 5A. IFN-γ production by the spleen cell cultures was monitored at 72 h as a measure of CD8⁺ T cell stimulation. IFN-γ production is indicated (Fig. 5B) for triplicate cultures ± SD in the absence of APC stimulation (no activation), in the presence of unloaded APCs (APC), and after stimulation with OVA peptide pulsed-APCs (APC + OVA-peptide). Spleen cells of OVA-*M*. *smithii*-archaeosome immunized mice responded to OVA₂₅₇₋₂₆₄ peptide stimulation by producing substantial IFN-γ (Fig. 5B). There was no non-specific activation in the absence of the SIINFEKL peptide. Similarly, spleen cells from mice immunized with OVA in the absence of adjuvant did not respond to peptide stimulation.

To evaluate whether CTL responses can be mediated by other archaeosome types as well, C57BU6 mice were immunized once (s.c.) with 15 µg OVA either in PBS alone (No adjuvant), or encapsulated in *M. smithii, T. acidophilum, H. salinarum,* or *M*. *stadtmanae* archaeosomes. Mice immunized with OVA encapsulated in *M. smithii* archaeosomes were used as a positive control. On day 14 spleens were harvested, pooled (n=3/group), and stimulated with irradiated EG.7 cells for 5 days, and then 4 h CTL activity on ⁵¹Cr labelled targets (EL-4 and EG.7) was assessed. % specific lysis ± SD of triplicate cultures is indicated (Fig. 6) at the various Effector:Target (E:T) ratios. All archaeosome types induced strong CTL responses (Fig. 6) after a single immunization.

In a further experiment, the CTL activity in spleens of mice receiving one (day 0) or two (days 0 and 21) OVA-archaeosome immunizations (s.c.) is compared. In this experiment, C57BL/6 mice were immunized as indicated in Figure 7, with 15 µg OVA either in PBS alone (No adjuvant), or encapsulated in *T*. *acidophilum* or *H. salinarum* archaeosomes. Spleens were harvested on day 28, stimulated with irradiated EG.7 cells, CTL activity assayed, and % specific lysis determined as described for Figure 6. Mice immunized once or twice with either of the OVA-archaeosomes demonstrated CTL activity, but the CTL activity of spleen cells of mice immunized twice was higher compared to mice receiving only one immunization with the corresponding archaeosome type (Fig. 7).

### Archaeosomes composed of purified archaeal lipids induce CTL activity

To illustrate the concept that archaeal polar lipids isolated in a biologically pure form, or polar lipid subfractions, may be substituted for the total polar lipids extract for archaeosome preparations of this invention, an experiment was performed as described in Table 1. OVA entrapped in archaeosomes composed of either archaetidyl glycerolphosphate-0-methyl or archaetidyl glycerol clearly induced CTL activity in mice.

### The CTL activity induced by archaeosomes is mediated by CD8⁺ T cells

As verification that the CTL activity induced by spleen cells of mice immunized with OVA-*M*. *smithii* archaeosomes was indeed mediated by CD8⁺ T cells, the experiment in Figure 8 was performed. C57BL/6 mice were immunized (i.p.) on days 0 and 21 with 15 µg OVA either in PBS alone (No adjuvant), or encapsulated in *M. smithii* archaeosomes. On day 28, the spleens were harvested, pooled (n=4/group) and stimulated with irradiated EG.7 cells for 5 days to multiply effectors. CD8⁺ T cells were depleted (eliminated) from an aliquot of M. *smithii* effectors *(M. smithii* -CD8 lysed) on day 5 with the use of anti-CD8 antibody and rabbit complement. Flow cytometric analysis of depleted effectors indicated the population to be > 99% CD8⁺ T cell depleted and there was no non-specific depletion of CD4⁺ T cells (data not shown). CTL activity of this preparation was compared to that of the positive (non-depleted *M. smithii* effectors) and negative (No adjuvant effectors) controls in a 4 h assay against ⁵¹Cr labelled targets (EL-4 and EG.7). Data represent % specific lysis ± SD of triplicate cultures at the various Effector:Target (E:T) ratios. Depletion of CD8⁺ T cells completely abrogated the lytic activity of OVA-M. *smithii* effectors towards EG.7 cells (Fig. 8), clearly demonstrating that the CTL response is CD8⁺ T cell mediated.

### CTL response induced by archaeosomes is CD4⁺ T cell Independent

To assess the role of CD4⁺ T cell help in the induction of the CTL responses by archaeosomes, normal control C57BL/6J mice and CD4⁺ T cell-deficient C57BL/6J mice were immunized once (s.c.) with 15 µg OVA encapsulated in *M. smithii* archaeosomes. Pooled spleen (n=2/group) cells obtained from both groups of immunized mice at 14 and 30 days after immunization were stimulated with EG.7 cells for 5 days *in vitro,* and CTL activity on ⁵¹Cr labelled targets assessed. % Specific lysis ± SD of triplicate cultures is indicated. Data in Fig. 9A and 9B demonstrate that after immunization with OVA-archaeosomes, CD4⁺ T cell deficient mice (CD4 -/-) evoked antigen-specific CTL activity comparable to that of the normal control mice. The CTL activity in the absence of CD4⁺ T cell help was evident even at 30 days post-immunization (Fig. 9B).

Several studies indicate that CD8⁺ T cell responses often require CD4⁺ T cell help for effective stimulation and long-standing immunity (Kalams and Walker, 1998). CD4⁺ T cells prevented the rapid depletion of CD8⁺ T cells after a transient response to antigen (Kirberg et al., 1993). Protective CD8⁺ T cell responses to chronic LCMV infection required CD4⁺ T cell help (Matloubian et al., 1994). Adjuvants composed of particulate viral antigens (virus-like particles) have also been shown to require CD4⁺ T cell help for evoking CD8⁺ T cell responses to exogenous antigen (Wild et al., 1999). CTL responses that result from cross priming, as after DNA vaccination, is also dependent on CD4⁺ T cell help (Maecker et al., 1998). Importantly, and in contrast to other observations above, archaeosomes effectively by-passed CD4⁺ T cell help for induction of strong CTL responses to entrapped antigen, even when using a single immunization with relatively low doses (15 µg) of the antigen. Thus, archaeosomes may be effective even in vaccines for immuno-compromised individuals such as AIDS patients.

### Archaeosomes induce long term CTL responses and memory

To determine the longevity of the CTL response induced, C57BL/6 mice were immunized (s.c., on days 0 and 21) with 25 µg OVA encapsulated in *M. smithii* archaeosomes. Representative mice (n=3 per time point) were terminated at regular intervals, the spleen cells re-stimulated with EG.7 cells for 5 days, and CTL activity assessed on ⁵¹Cr labelled EL-4 and EG.7 targets. Data are represented as lytic units ± SD of spleen cells from individual mice. Killing of EL-4 targets was < 5 % even at 100:1, effector:target ratio for all time points tested. The results (Fig. 10) demonstrate that OVA-archaeosomes induce a strong recall (memory) CTL response that dramatically increases over a period of time and is maximal even at 150 days post first immunization. Similar trends were obtained when data were expressed as lytic units per whole spleen (data not shown). Thus, delivery of soluble antigens entrapped in archaeosomes not only evokes a CTL response in the short term, but also induces potent CD8⁺ T cell memory.

Memory T cells are characterized by the high expression of cell surface molecules such as CD44, and are usually more responsive to antigen challenge. To establish the development and activation of memory cells, representative OVA-*M*. *smithii* archaeosome immunized mice (n=2) from experiments described in Figure 10 and naïve control mice (n=2) were challenged by injecting OVA₂₅₇₋₂₆₄, CTL epitope-pulsed IC21 macrophages (10 X 10⁸ cells, given i.p.) on day 140. Five days later, the spleen cells were analyzed for the expression of CD44, LFA-1 (CD11a) and CD28 by flow cytometry. Expression of cell surface markers is indicated for gated, splenic CD8⁺ T cell populations from control (naïve) and OVA-*M*. *smithii* archaeosome immunized mice (Fig. 11). Data from 25,000 events were analyzed. Numbers within each panel indicate the percentage of CD8⁺ T cells staining for each marker. Compared to the naïve controls (top panel, Fig. 11), a dramatic upregulation (~ 3 fold) of CD44 was seen in the case of OVA-archaeosome immunized mice (lower panel, Fig.11). This induction of CD44 expression in OVA-archaeosome immunized mice was antigen-specific, since boosting with IC21 cells without the peptide did not enhance the expression (data not shown). As CD44^{hi} cells are classically associated with the memory phenotype (Dutton et al., 1998), the strong upregulation of CD44 observed even 140 days post immunization using a relatively low antigen dose (25 µg/injection), indicates the establishment of a potent memory response. The modulation of other cell surface molecules such as LFA1 and CD28 also suggests efficient activation and responsiveness of the memory CD8⁺ T cells to antigen challenge.

### Archaeosomes activate APCs

J774A.1 macrophages (10⁵/ml) were incubated for 24 h with *M. smithii* archaeosomes (25 µg/ml lipid), conventional liposomes (25 µg/ml lipid), or LPS (10 µg/ml lipid) to assess *in vitro* activation of APCs. Cells were then washed, recovered, and double-stained with Mac 1α-PE and one other FITC-labeled cell surface activation marker as indicated, and analyzed by flow cytometry (Fig.12). Data from 20,000 events were analyzed. The hashed line indicates the positive stain for each activation marker. Numbers within each panel indicate the percentage of Mac 1α⁺ cells staining for each marker in the various treatment groups. Non-activated J774A.1 cells constitutively showed a moderate expression of costimulatory marker CD86 (B7.2) and a strong expression of MHC class 1 molecules, but failed to express MHC class II and CD80 (B7.1). Exposure of J774A.1 cells to archaeosomes led to strong upregulation of CD80 (B7.1), CD86 (B7.2) and MHC class II molecules. The expression levels of various markers after archaeosome treatment were often similar to those obtained by treating cells with LPS, a known activator of macrophages. In contrast, J774A.1 cells exposed to a comparable amount of conventional liposomes expressed levels of costimulatory activation markers and MHC class II molecules similar to the non-activated, negative-control macrophages (Fig. 12).

Similar immunomodulation to that measured with J774A.1 cells was obtained when bone marrow derived dendritic cells (DCs) were exposed to archaeosomes. Bone marrow derived dendritic cells (10⁵/ml) were cultured for 24 h in the absence (no activation) or presence of *M. smithii* archaeosomes (25 µg lipid/ml). Cells were then washed, recovered, and single stained with anti-mouse Ia^{d} (MHC class II), B7.1, B7.2 or appropriate isotype-specific antibodies, and analyzed by Flow cytometry. Data profiles were obtained after analysis of 20,000 events. Figure 13A profiles indicate the staining of non-activated bone marrow DCs, and Figure 13B of the archaeosome treated DCs, for the indicated markers. The profile shown as a hashed line indicates the negative staining (with the isotype-specific antibody), and the solid line profile the positive staining (with the specific antibody). The percentages within each panel indicate the number of cells staining strongly for each marker in the two groups. It is seen that non-activated DCs strongly express MHC class II molecules. However, incubation of these DCs with archaeosomes led to even further upregulation of MHC class II expression. Further, there was also a strong upregulation of B7.2 expression after archaeosome treatment. Thus, archaeosomes induced a rapid and potent upregulation of MHC class II and costimulatory molecules on APCs.

We next show that archaeosomes injected into the peritoneal cavities of mice activate APCs *in vivo.* BALB/c mice (n=3) were injected i.p. with 1 mg of *M*. *smithii* archaeosomes (in 200 µl final volume of PBS), or with 200 µl PBS (PBS control). Peritoneal cells were recovered 5 days later and stained with anti-mouse Ia^{d}-FITC labeled antibody (MHC class II), and analyzed by Flow cytometry. The MHC class II staining (as a representative activation marker) of peritoneal exudate cells as deduced from 20,000 events is indicated by the profiles in Figure 14. The results highlight the strong upregulation of MHC class II expression in cells obtained from archaeosome treated mice (solid line expression profile), as compared to cells from the PBS controls (hashed line expression profile).

Activated antigen presenting cells often exhibit enhanced capacity to secrete inflammatory cytokines, particularly tumor necrosis factor-α (TNF). We therefore tested if treatment of APCs with archaeosomes also led to enhanced secretion of TNF. J774A.1 cells (Fig. 15A) or bone-marrow DCs (Fig. 15B), were incubated *in vitro* with varying concentrations of conventional liposomes (Con. Liposomes), or *M. smithii* archaeosomes for 48 h, and TNF produced in the supernatant determined as described in Materials and Methods. TNF production is indicated as mean± SD of triplicate cultures. Sensitivity of the TNF assay was <0.1 pg/ml. Macrophages (J774A.1 cells) or dendritic cells that had been incubated with *M*. *smithii* archaeosomes produced substantial amounts of TNF, whereas conventional liposomes failed to invoke significant increases in TNF production (Fig.15A and 15B).

### Archaeosomes facilitate recruitment of Mac 1α positive cells in vivo

To decipher the effects of archaeosomes on cell populations *in vivo,* we analyzed the recruitment of APC populations into the peritoneum of mice, after i.p. injection of empty archaeosomes. BALB/c mice (n=2) were injected (i.p.) with 1 mg/200 µl *M*. *smithii* archaeosomes, or 200 µl of PBS. At 4 and 14 days after injection, cells were recovered by peritoneal lavage, and analyzed for the expression of Mac1α. Representative data in Figure 16 shows the Mac 1 a expression profile of peritoneal exudate cells from control (PBS-treated) and archaeosome-treated mice. Data are deduced from 20,000 events for each sample. Three distinct populations can be indentified based on Mac 1α staining: Mac 1α⁻, Mac 1α^{low}, and Mac 1α^{hi}, particularly in the archaeosome-treated mice. The percentage of these three populations in the peritoneum at various time points after archaeosome injection is also indicated in Figure 16. It is evident that in control mice the primary cell population is Mac 1α⁻. PBS control mice exhibited similar cell profiles on all days post-injection. In contrast, archaeosome treated mice show a decrease in the Mac 1α⁻ cells as early as day 2 (not shown) and 4, and a concomitant increase in Mac1α⁺ cells. By day 14 of archaeosome treatment, Mac 1α^{hi} cells become the predominant population, accounting for > 60 % of the cells in the peritoneum. As Mac 1α expression is seen primarily on cells of the monocytic lineage (including macrophages and dendritic cells), these results show that archaeosomes facilitate the sustained recruitment of these cell populations into the injection site.

To further characterize the Mac 1α^{hi} cell population that was recruited and activated by archaeosomes *in vivo,* BALB/c mice were injected i.p. with 1 mg of *M*. *smithii* archaeosomes in 200 µl of PBS. On day 5, peritoneal exudate cells were analyzed for the expression of Mac 1α (Fig. 17A). Based on Mac1α expression, as indicated in the figure, cells were sorted into Mac1α ^{low} and Mac1α^{hi} populations (Fig. 17B). The purity of the sorted populations is represented by the histograms. The sorted cells (2 X 10⁵/ml) were cultured *in vitro* for 48 h with GM-CSF (5 ng/ml). Cultured cells were then recovered, washed and analyzed for the expression of F4/80, CD11c, and B220 (Fig. 17C). All histograms are deduced from the analysis of 20,000 events.

Interestingly, the Mac1α^{hi} cells gave rise to two populations expressing F4/80 or CD11c (Fig. 17C, right panel). Since strong F4/80 expression is associated with activated macrophages, and CD11c expression with mature dendritic cells (Pulendran et al., 1997), it is clear that the Mac 1α^{hi} cells recruited and activated by archaeosomes *in vivo,* included precursors of both these potent APC populations. Mac 1α^{low} cells on the other hand, stained negative for F4/80, and CD11c, and were found to be primarily B220⁺ cells (Fig. 17C, left panel).

### Archaeosome-activated APCs stimulate T cells

In this example we show that *in vitro* activation of APCs (macrophages and DCs) by archaeosomes translates into an increased functional ability of the APCs to stimulate T cell proliferation. H-2K^{d} halotype J774A.1 cells or bone marrow derived dendritic cells (10⁵/ml), were incubated with conventional liposomes (25 µg lipid/ml), or archaeosomes (*M. smithii,* 25 µg lipid/ml), or LPS (10 µg/ml) for 24 h and cells were subsequently washed, recovered and used as APCs for stimulating allo-specific T cells. Purified allo-specific (H-2K^{b}) CD8⁺ T cells were stimulated with 2 X 10⁴ J774A.1 cells (Fig. 18A). Alternatively, purified allo-specific CD4⁺ T cells were stimulated with 10⁴ DCs (Fig. 18B). At 72 h the cells were pulsed with ³H thymidine and proliferation assessed 18 h later by liquid scintillation counting. Data represent the mean counts per min (CPM) ± standard deviation of triplicate cultures.

Macrophages (J774A.1, H-2K^{d}) exposed to archaeosomes strongly stimulated the proliferation of purified allo-specific (H-2K^{b}) CD8⁺ T cells (Fig. 18A). This proliferation was comparable to that achieved with LPS-activated macrophages. In the absence of any activation, J774A.1 APCs induced only a modest CD8⁺ T cell proliferation. Similarly, non-activated bone marrow derived DCs (H-2K^{d}) showed low ability to induce the proliferation of purified naïve allo-specific (H-2K^{b}) CD4⁺ T cells. However, prior activation of dendritic cells with archaeosomes, led to strong proliferation of the T cells (Fig. 18B). In contrast, dendritic cells exposed to conventional liposomes failed to enhance T cell stimulation.

### APCs Induced in vivo by archaeosomes strongly stimulate T cell proliferation and cytokine production

Having demonstrated the ability of archaeosomes to recruit and activate APCs *in vivo,* we evaluated the ability of these APCs to stimulate T cells. BALB/c mice (n=3) were injected (i.p.) with 1 mg/200 µl *M*. *smithii* archaeosomes, or 200 µl of PBS and 5 days later the peritoneal exudate cells were recovered. Cells (10⁴) were washed, counted, irradiated and used as APCs to stimulate allo-specific (H-2K^{b}) purified CD4⁺ and CD8⁺ T cells. The proliferation of the T cells was monitored by ³H thymidine incorporation at 72 h (Fig. 19A). IFN-γ production in the supernatant at 72 h was assayed by ELISA (Fig. 19B). Data represent mean ± SD of values from triplicate cultures. Peritoneal APCs from archaeosome-treated mice induced strong proliferation of both CD4⁺ and CD8⁺ T cells (Fig. 19A). Furthermore, the T cells stimulated by archaeosome-activated APCs produced substantial IFN-γ (Fig.19B). In contrast, peritoneal exudate cells from control mice (treated with PBS alone) were unable to induce T cell proliferation or cytokine production.

### Protective vaccine for Listeria monocytogenes

Immunity against intracellular pathogens depends upon the generation and subsequent actions of pathogen-specific CD8⁺ T cells. These lymphocytes are best generated by prior exposure to a sublethal dose of the live pathogen or by vaccination with a live attenuated version of the pathogen (Zhan and Cheers, 1998). This invention contributes towards a major goal of modem vaccinology; namely to replace the need for live organisms with defined acellular vaccines which generate protective immunity against intracellular pathogens. A key to realizing such vaccines appears to be to develop antigen delivery strategies that emulate the means by which antigens expressed on live organisms are exposed to the host immune system.

*Listeria monocytogenes* is a prototypical intracellular pathogen, and systemic infection of mice with this organism has been used for over four decades as a model for studying cell mediated immunity. *L. monocytogenes* is capable of parasitizing epithelial cells, professional phagocytes, and parenchymal cells. Antibodies afford absolutely no defence against this pathogen. It is generally agreed that protective immunity against murine listeriosis is almost entirely mediated by CD8⁺ T cells, as opposed to CD4⁺ T cells (North and Conlan, 1998). The precise function of CD8⁺ T cells most probably involves macrophage activation as well as cytolysis of infected cells. For examining protective CD8⁺ T-cell-mediated immunity, the utility of this infection model is beyond dispute.

We therefore examined the ability of archaeosome based vaccines to elicit protective immunity against this pathogen, as an example illustrative for protective vaccinations against intracellular pathogens generally. A nonamer peptide of the *L*. *monocytogenes* haemolysin, listeriolysin, has been shown to be a highly immunodominant MHC Class I restricted CD8⁺ T-cell-specific epitope for BALB/c mice (Vijh and Pamer, 1997). We prepared two bipalmitoylated peptides containing the aforementioned immunodominant nonameric amino acid sequence for encapsulating into archaeosomes and determining the efficacy of these vaccines in murine model of infection.

### Archaeosome-peptide vaccination induces antigen-specific CD8⁺ T cell response

BALB/c mice were vaccinated twice (s.c.), 28 days apart, with either the free (bare) 20-mer lipopeptide (27 µg) or with the 20-mer lipopeptide (27 µg) encapsulated in *M*. *smithii* archaeosomes (1 mg lipid). Three weeks after the second immunization, 2 mice each from naïve control and the two vaccinated groups were taken for immunological analysis.

In one experiment, splenocytes from each group of mice were stimulated *in vitro* for 72 h with the indicated amounts of the specific listeriolysin nonamer peptide, and antigen-specific induction of IFN-γ secretion (± SD for triplicate cultures) into the cell supernatant assessed (Fig. 20A). Splenocytes from mice vaccinated with peptide encapsulated in *M. smithii* archaeosomes, but not those from naïve control (unvaccinated) mice, or mice vaccinated with 20-mer lipopeptide alone, responded to exposure to the listeriolysin-nonamer peptide as seen by secreting substantial quantities of IFN-γ. This indicated that the lipopeptide encapsulated in archaeosomes is capable of potentiating an antigen-specific CD8⁺ T cell response.

In a parallel experiment, bulk cultures of the splenocytes were stimulated for 5 days by co-incubation with irradiated Phem 3.3 cells (derivative of P815 cells transfected with the gene for listeriolysin). The effector T cells thus generated were tested for specific cytotoxicity against ⁵¹Cr-labelled Phem 3.3 or P815 cells using a standard chromium release assay (Fig. 20B). % specific lysis ± SD for triplicate cultures, using a range of effector to target ratios (E:T) is shown. Splenocytes from mice vaccinated with lipopeptide entrapped in archaeosome killed the transfected Phem 3.3 cells, but not parental P815 cells not expressing the peptide. In contrast to this, splenocytes from naïve mice, or mice immunized with the free (non-encapsulated) peptide failed to evoke a measurable CTL response as judged by their failure to lyse Phem 3.3 cells.

Based on the results above, we evaluated the efficacy of archaeosomes containing encapsulated lipopeptide to confer protection against pathogen challenge (Table 2). BALB/c mice vaccinated with lipopeptide encapsulated in archaeosomes had 8 to 38-fold fewer *Listeria* in their livers and at least 380 to 2042-fold fewer *Listeria* in their spleens than was found in corresponding organs in any of the control groups, which included unvaccinated mice (naïve mice), mice vaccinated with either the lipopeptide alone or the archaeosomes alone, and mice immunized with preformed empty archaeosomes admixed with the lipopeptide. A 1 log₁₀ reduction in CFU represents a 90% reduction of pathogen burden, and the difference between a 1 log₁₀ and a 3 log₁₀ reduction in bacterial burden only represents the difference between a 90% and a 99.9% reduction in bacterial numbers. Viewed in this light, there is probably little biologically-meaningful difference between the immunity expressed in the liver and the spleen. The immunity elicited by vaccination with the lipopeptide encapsulated in archaeosomes was antigen-specific, because mice immunized with lipopeptide-archaeosome were found to be no more resistant than unvaccinated control mice to an intravenous challenge with the heterologous facultative intracellular bacterium, *Salmonella typhimurium.* Thus there is no prolonged non-specific enhancement of antibacterial resistance following injection of listeria antigen entrapped in archaeosomes.

Using the murine model of systemic listeriosis, we have demonstrated the potential of archaeosomes as immunomodulating, antigen-presenting vehicles for subunit vaccines capable of eliciting protective cell mediated immunity. No augumentation of the preparation was required beyond encapsulation of the antigen. In contrast, others have reported that conventional liposomes require co-entrapment of the immunostimulant, Quil-A, with the listeriolysin peptide antigen, to elicit protection (Lipford et al., 1994a). Usually, anti-*Listeria* immunity can be generated in mice following exposure to a sublethal infection with virulent *L*. *monocytogenes,* or listeriolysin-producing mutants with attenuated virulence. By contrast, vaccinating mice with viable listeriolysin-negative bacteria, or killed virulent bacteria, or *Listeria*-derived proteins fails to generate such protective immunity (Barry et al., 1992; Zhan and Cheers, 1998). Recently, other researchers have reported success in generating protective immunity by vaccinating mice with killed *L*. *monocytogenes* admixed with the cytokine, interleukin-12 (Miller et al., 1996). However, nearly all of this protection appears to be due to nonspecific activation of the immune system by the exogenous IL-12, and as such will undoubtedly be short-lived. Moreover, IL-12 is known to be highly toxic for the host. Vaccination of mice with the listeriolysin peptide antigen or whole listeriolysin, when incorporated into live bacterial and viral vectors generates protective immunity against challenge with virulent *Listeria monocytogenes* (Ann et al. 1996; Gentshev et al., 1996; Sirard et al., 1997), but these suffer the drawbacks of live vaccines mentioned earlier. The listeriolysin peptide antigen fused with anthrax toxin as a carrier molecule also acts as an anti-*Listeria* vaccine (Ballard et al., 1996). However, a major drawback of this approach is that the vaccine carrier itself is immunogenic. This reduces its utility, since it cannot be used repeatedly. The results of the current invention are novel and contrary to prior art since archaeosomes act as immunomodulating carriers for non replicating peptide antigens, without the need for additional immunostimulants such as Quil A or IL 12, or the need to use viable, replicating carriers for the antigen.

### Persistence of anti-Listeria immunity

Data were shown earlier to support the longevity and memory immunological responses generated in animals to archaeosome-delivered antigens. Next the persistence of anti*-Listeria* immunity in mice that were vaccinated with the lipopeptide encapsulated in *M. smithii* archaeosomes was evaluated by challenging the vaccinated mice with *L*. *monocytogenes* at up to 10 months after the final vaccination (Table 3). The level of protective immunity seen after 1 month post-vaccination (Table 2) persisted at least for 10 months post-vaccination. Archaeosome-based anti-*Listeria* vaccines used herein appear to elicit superior longer-term protective immunity than the anthrax-toxin-based fusion vaccine (Ballard et al., 1996), the recombinant vaccinia vaccine (Ann et al., 1996), and the naked DNA-based vaccines (Cornell et al., 1999). None of the other vaccination strategies (Lipford et al., 1994a; Ann et al., 1996; Ballard et al., 1996) demonstrated the ability to elicit the rapidly expressed immunity that archaeosome-based vaccines are clearly capable of inducing.

### Kinetics of expression of anti-Listeria immunity

Next, the kinetics of the appearance of enhanced anti-*Listeria* resistance in immunized mice was examined (Fig.21A and 21B). Groups of BALB/c mice were vaccinated (100 µl injection volume) on days 0, 21, 42 with 12.5 µg of the 20-mer lipopeptide antigen either in PBS alone (●), or encapsulated in 0.5 mg of *M. smithii* archaeosomes (o). Six weeks after the last vaccination, mice were challenged with an intravenous inoculum of 3.18 x 10³ CFU of *L. monocytogenes.* Bacterial burdens were determined in the livers (Fig. 21A), and spleens (Fig. 21 B) of both groups of mice 1, 2, and 3 days after challenge. Mice vaccinated with antigen entrapped in *M. smithii* archaeosomes expressed anti*-Listeria* immunity in the spleens as early as 24 h after infection, and in the livers after 48 h of infection.

### Rapid onset of immunity following vaccination with antigen entrapped in various archaeosomes

To assess the time of onset of anti-*Listeria* resistance following immunization, mice were vaccinated once with the listeriolysin-derived 20-mer lipopeptide entrapped in archaeosomes prepared from either *M*. *smithii* or *H*. *salinarum,* and challenged with *L. monocytogenes* 7 days later. Bacterial burdens in the spleen and liver were determined on day 3 of infection. The results in Table 4 show that substantial anti*-Listeria* resistance was expressed in both organs of mice vaccinated with either lipopeptide-archaeosome formulation. This resistance was specific (not innate immunity), because it was absent in mice vaccinated with empty archaeosomes.

The 20-mer lipopeptide antigen, delivered in archaeosomes prepared from the total polar lipids of extreme halophiles (*H. salinarum, N. magadii),* a thermoacidophile (*T*. *acidophilum*), or the methanogen, *M. smithii,* elicits superior early protective immunity than that elicited by the same antigen entrapped in conventional ester phospholipid liposomes (Table 5). This immunity appeared to be antigen-specific, because mice vaccinated with antigen entrapped in archaeosomes prepared from *H. salinarum* were no more resistant than control mice to a challenge with *S*. *typhimurium.* Moreover, in a complementary study (Table 6), the early immunity expressed following vaccination with antigen entrapped in *T. acidophilum* archaeosomes was found to be haplotype-restricted and pathogen-specific.

When groups of BALB/c mice immunized once, as in Table 5, were challenged one month post-vaccination, all archaeosome formulations displayed similar levels of protective immunity (Table 7).

In another example, groups of twice-vaccinated mice were challenged 1 month later with a 10-fold larger dose *of L. monocytogenes.* The protection in the spleen elicited by vaccination with antigen entrapped in *M. smithii* and *T. acidophilum* archaeosomes was superior to that induced by the antigen encapsulated in either *H*. *salinarum* archaeosomes, or conventional liposomes. However, in the liver, immunization with any of the three archaeosome types resulted in lower CFUs compared to conventional liposome immunizations (Table 8).

### Antigen and archaeosome dose required to confer rapid protection in the L. monocytogenes model

For these studies the 13-mer lipopeptide was entrapped in *T*. *acidophilum* archaeosomes. Mice were immunized with the vaccine that was diluted serially before injection, thus maintaining a constant antigen to lipid ratio. One week later protection was assessed by challenging the animals with the live pathogen. The data reveal that rapid protection occurred after a single immunization at all doses of the *T*. *acidophilum* archaeosome vaccine, varying from 23 µg lipopeptide in 500 µg archaeosomes to as little as 0.5 µg lipopeptide in 10 µg archaeosomes (Table 9).

### Francisella tularensis vaccine

*Francisella tularensis* is a Gram-negative, facultative intracellular pathogen. It is the etiological agent of tularemia, a severe and often fatal disease of humans and other mammals. Because *F*. *tularensis* is an intracellular pathogen that can reside in both macrophages and parenchymal cells, cell-mediated rather than humoral immunity is thought to be required to combat it. Clinically this is supported by the finding that immunization of laboratory personnel with an experimental attenuated live vaccine, but not with a killed whole-cell vaccine, imparts protective immunity. Comprehensive studies using murine models of tularemia have convincingly demonstarted that specific T-cell-mediated immunity is key to the efficient resolution of both primary and secondary *F*. *tularensis* infections (Conlan et al., 1994).

We therefore used tularemia as another model of intracellular parasitism to test the capacity of archaeosomes to act as immunomodulating carriers for non-replicating vaccines against intracellular pathogens. The antigen used was an outer membrane preparation isolated from *F. tularensis.* The isolated outer membranes were characterized with respect to size using a particle sizer, and were vesicles of 65 ± 37 nm diameter. To assess whether a fusion product between outer membranes and archaeosomes had been made, samples of outer membranes, *M*. *smithii* archaeosomes, and the putative fusion product between outer membranes and *M*. *smithii* archaeosomes were separately mixed with one volume of Percoll^{®} (Pharmacia Fine Chemicals) and two volumes of PBS. Samples were then centrifuged at 7,700 x g for 1.5 h. Empty archaeosomes (no outer membranes) formed a band near the surface of the gradient, whereas outer membranes formed a single discrete band several centimeters into the gradient. Evidence for a fusion product appeared as a disappearance of the outer membrane and archaeosome bands, and formation of a new band of intermediate density.

Mice immunized twice with the aforementioned fusion product showed protection against an aerosol challenge with the virulent pathogen (Table 10). In some cases tissues were sterile, despite the small antigen dose given.

### Protection against tumor development in mice pre-immunized with archaeosome-antigen

EG.7 is a T cell lymphoma cell line that has been transfected with the gene encoding OVA, and is known to induce solid tumors in mice. Groups of C57BL/6 mice were immunized s.c. on days 0 and 21 with empty archaeosomes (260 µg *T. acidophilum* or 312 µg *M. smithii* archaeosomes), or with 15 µg OVA in PBS, or with 15 µg OVA encapsulated in the respective amounts of archaeosomes above. Another group of mice was immunized with 25 µg OVA encapsulated in 1 mg of *H*. *salinarum* archaeosomes. Naïve (control) mice received no immunizations. All mice were challenged 8 weeks post first injection with 10x10⁶ EG.7 tumor cells, and tumor size measured, as described in Materials and Methods. All mice in the naïve group (Fig. 22A), OVA alone immunized group (Fig. 22B), and empty archaeosomes immunized group (Fig. 22D and F) developed tumors. However, outstanding protection against tumor growth and development occurred in mice pre-immunized with antigen entrapped in *H. salinarum* (Fig. 22C), *T*. *acidophilum* (Fig. 22E), or *M. smithii* (Fig. 22G) archaeosomes. The progression of tumor size in each mouse is plotted against time, in Figures 22A-22G.

The protective effect of archaeosome-antigen immunization was also apparent in another model of potent metastatic tumor. B16-OVA is a melanoma cell line of H-2^{b} origin that has been transfected with the gene encoding OVA, and is known to induce metastatic tumor foci in the lungs 14 days after tumor challenge. The experiment was conducted as described under Materials and methods. Naïve mice injected with these tumor cells intravenously developed > 100 black metastatic foci in the lungs 14 days after tumor challenge. In contrast, two out of the three OVA-*M*. *smithii* archaeosome immunized mice had dramatically reduced lung foci (8 and 20, respectively), and only one exhibited foci number comparable to the naïve control mice, upon tumor challenge.

### Therapeutic effect of archaeosomes against tumors

The therapeutic effect of empty archaeosomes, and of archaeosomes containing encapsulated antigen, on tumor growth was evaluated as follows. C57BL/6 mice were first injected (s.c.) with 10x10⁶ EG.7 tumor cells, followed by immunization on days 0 and 10 with nothing (naïve), or 15 µg OVA, or 15 µg OVA encapsulated in 144 µg of either *T*. *acidophilum* or *M. smithii* archaeosomes, or with 144 µg of either type of empty archaeosomes. Injections of OVA alone had no influence on tumor growth/progression (compare Fig. 23A and 23B). Injecting empty archaeosomes of *T*. *acidophilum* resulted in complete regression (clearing) of tumors in 2 of 5 mice (Fig. 23C), and showed similar complete tumor regression (2/5 mice) and prevented formation of large tumors in the remainder when OVA antigen was encapsulated in the respective archaeosome (Fig. 22D). Empty archaeosomes of *M*. *smithii* had an especially strong therapeutic effect, regressing tumors in 5 of 5 mice (Fig. 23E).

### REFERENCES

1. Ann, L.L., Pamer, E., and Whitton, J.L. (1996) A recombinant minigene vaccine containing a nonameric cytotoxic T-lymphocyte epitope confers limited protection against Listeria monocytogenes infection. Infect. Immun. 64: 1685-1693.
2. Ballard, J.D., Collier, R.J., and Starnbach, M.N. (1996) Anthrax toxin-mediated delivery of a cytotoxic T-cell epitope in vivo. Proc. Natl. Acad. Sci. USA. 93: 12531-12534.
3. Barbier, J.-R., Neugebauer, W., Morley, P., Ross, V., Soska, M., Whitfield, J.F., and Willick, G. (1997) Bioactivities and secondary structures of constrained analogues of human parathyroid hormone: Cyclic lactams of the receptor binding region. J. Med. Chem. 40: 1373-1380.
4. Barry, R.A., Bouwer, H.G., Portnoy, D.A., and Hinrichs, D.J. (1992) Pathogenicity and immunogenicity of Listeria monocytogenes small-plaque mutants defective for intracellular growth and cell-to-cell spread. Infect. Immun. 60: 1625-1632.
5. Bowersock, T.L., and Martin, S. (1999) Vaccine delivery to animals. Advanced Drug Delivery Rev. 38: 167-194.
6. Cherwinski, H.M., Schumacher J.H., Brown, K.D., and Mosmann, T.R. (1987) Two types of mouse helper T cell clone. III. Further differences in lymphokine synthesis between Th1 and Th2 clones revealed by RNA hybridization, functionally monospecific bioassays, and monoclonal antibodies. J. Exp. Med. 166: 1229-1244.
7. Choquet, C.G., Patel, G.B., Beveridge, T.J., and G.D. Sprott. (1994) Stability of pressure-extruded liposomes made from archaeobacterial ether lipids. Appl. Microbiol. Biotechnol. 42: 375-384.
8. Conlan, J.W. (1997) Critical roles of neutrophils in host defense against experimental systemic infections in mice by Listeria monocytogenes, Salmonella typhimurium, and Yersinia enterocolitica. Infect. Immun. 65: 630-635.
9. Conlan, J.W., Sjostedt, A., and North, R.J. (1994) CD4+ and CD8+ T-cell-dependent and-independent host defense mechanisms can operate to control and resolve primary and secondary Francisella tularensis LVS infection in mice. Infect. Immun. 62: 5603-5607.
10. Cornell, K.A., Bouwer, H.G.A., Hinrichs, D.J., and Barry, R.A. (1999) Genetic immunization of mice against Listeria monocytogenes using plasmid DNA encoding listeriolysin O. J. Immunol. 163: 322-329.
11. Dutton, R.W., Bradley, L.M., and Swain, S.L. (1998) T cell memory. Annu. Rev. Immunol. 16: 201-223.
12. Fukasawa, M., Shimizu, Y., Shikata, K., Nakata, M., Sakakibara, R., Yamamoto, N., Hatanaka, M., and Mizuochi, T. (1998) Liposome oligomannose-coated with neoglycolipid, a new candidate for a safe adjuvant for induction of CD8+ cytotoxic T lymphocytes. FEBS Letters, 441: 353-356.
13. Gentshev, I., Mollenkopf, H., Sokolovic, Z., Hess, J., Kaufmann, S.H., and Goebel, W. (1996) Development of antigen-delivery systems based on the Escherichia coli hemolysin secretion pathway. Gene 179: 133-140.
14. Gupta, R.K., Rost, B.E., Reyveld, E., and Siber, G.S. (1995) Adjuvant properties of aluminum and calcium compounds. In: M.F. Powell and M.J. Newman, (ed.), Vaccine design, the subunit and adjuvant approach, p. 229-248. Plenum Press, New York.
15. Harokopakis, E., Hajishengallis, G., and Michalek, S.M. (1998) Effectiveness of liposomes possessing surface-linked recombinant B subunit of cholera toxin as an oral antigen delivery system. Infect. Immun. 66: 4299-4304.
16. Henkart, P.A. (1997) CTL effector functions. Semin. Immunol. 9: 85-86.
17. Kalams, S.A., and Walker, B.D. (1998) The critical need for CD4 help in maintaining effective cytotoxic T lymphocyte responses. J. Exp. Med. 188: 2199-2204.
18. Kates, M. (1992) Archaebacterial lipids: structure, biosynthesis and function. Biochem. Soc. Symp. 58: 51-72.
19. Kates, M., Moldoveanu, N., and Stewart, L.C. (1993) On the revised structure of the major phospholipid of Halobacterium salinarum. Biochim. Biophys. Acta 1169: 46-53.
20. Kirberg, J., Bruno, L., and von Boehmer, H. (1993) CD4+8- help prevents rapid deletion of CD8+ cells after a transient response to antigen. Eur. J. Immunol. 23: 1963-1967.
21. Krishnan, L. and Mosmann, T.R. (1998) Functional subpopulation of CD4+T lymphocytes.p. 7-32. In: I. Kimber and M.K. Selgrade, (ed.), T lymphocyte subpopulations in immunotoxicology. John Wiley & Sons, Chichester.
22. Leslie, D.L., Cox ,J., Lee, M., and Titball, R.W. (1993) Analysis of a cloned Francisella tularensis outer membrane protein gene and expression in attenuated Salmonella typhimurium. FEMS Microbiol. Lett. 111: 331-336.
23. Lipford, G.B., Lehn, N., Bauer, S., Heeg, K., and Wagner, H. (1994a) The immunodominant peptide from listeriolysin in Quil A liposomes vaccinates CD8+ cytolytic T cells and confers protection to infection. Immunol. Letts. 40:101-104.
24. Lipford, G.B., Wagner, H., and Heeg, K. (1994b) Vaccination with immunodominant peptides encapsulated in Quil A-containing liposomes induces peptide-specific primary CD8+ cytotoxic T cells. Vaccine, 12: 73-80.
25. Maecker, H.T., Umetsu, D.T., DeKruyff, R.H., and Levy, S. (1998) Cytotoxic T cell responses to DNA vaccination: dependance on antigen presentation via class II MHC. J. Immunol. 161: 6532-6536.
26. Matloubian, M., Concepcion, R.J., and Ahmed, R. (1994) CD4+ T cells are required to sustain CD8+ cytotoxic T-cell responses during chronic viral infection. J. Virol. 68: 8056-8063.
27. Miller, M.A., Skeen, M.J., and Ziegler, H.K. (1996) Protective immunity to Listeria monocytogenes by immunization with heat-killed Listeria and IL-12. Ann. N.Y. Acad. Sci. 797: 207-227.
28. Mosmann, T.R., and Fong, T.A. (1989) Specific assays for cytokine production by T cells. J. Immunol. Methods 116: 151-158.
29. North, R.J., and Conlan, J.W. (1998) Immunity to Listeria monocytogenes. Chem. Immunol. 70: 1-20.
39. Ohara, J., and Paul, W.E. (1985) Production of a monoclonal antibody to and molecular characterization of B-cell stimulatory factor-1. Nature, 315: 333-336.
30. Pamer, E.G., Harty, J.T., and Bevan, M.J. (1991) Precise predicition of a dominant class I MHC-restricted epitope of Listeria monocytogenes. Nature (Lond.) 353: 852-855.
31. Pulendran, B., Lingappa, J., Kennedy, M.K., Smith, J., Teepe, M., Rudensky, A., Maliszewski, C.R., and Maraskovy, E. (1997) Development of pathways of dendritic cells in vivo: distinct function, phenotype, and localization of dendritic cell subsets in FLT3 ligand-treated mice. J. Immunol. 159: 2222-2231.
32. Richards, R.L., Rao, M., Wassef, N.M., Glenn, G.M., Rothwell, S.W., and Alving, C.R. (1998) Liposomes containing lipid A serve as an adjuvant for induction of antibody and cytotoxic T-cell responses against RTS,S malaria antigen. Infect. Immun. 66: 2859-2865.
33. Rotzschke, O., Falk, K., Stevanovic, S., Jung, G., Walden, P., and Rammensee, H. G. (1991) Exact prediction of a natural T cell epitope. Eur. J. Immunol. 21: 2891-2894.
34. Sad, S., Marcotte, R., and Mosmann, T.R. (1995) Cytokine-induced differentiation of precursor mouse CD8+ T cells into cytotoxic CD8+ T cells secreting Th1 and Th2 cytokines. Immunity, 2: 271-279.
35. Sirard, J.C., Fayolle, C., de-Chastellier, C., Mock, M., LeClrec, C., and Berche, P. (1997) Intracytoplasmic delivery of listeriolysin O by a vaccinal strain of Bacillus anthracis induces CD8-mediated protection against Listeria monocytogenes. J. Immunol. 159: 4435-4443.
36. Sjolander, A., Cox, J.C., and Barr, I.G. (1998) ISCOMs: an adjuvant with multiple functions. J. Leukoc. Biol. 64: 713-723.
37. Vijh, S., and Pamer, E.G. (1997) Immunodominant and subdominant CTL responses to Listeria monocytogenes infection. J. Immunol. 158: 3366-3371.
38. Wessel, D., and Flugge, U.I. (1984) A method for the quantitative recovery of protein in dilute solution in the presence of detergents and lipids. Anal. Biochem. 138:141-143.
39. White, W.I., Cassatt, O.R., Madsen, J., Burke, S.J., Woods, R.M., Wassef, N.M., Alving, C.R. and Koenig, S. (1995) Antibody and cytotoxic T-lymphocyte responses to a single liposome-associated peptide antigen. Vaccine, 13: 1111-1122.
40. Wild, J., M. Grusby, J., Schirmbeck, R., and Reiman, J. (1999) Priming MHC-I restricted, cytotoxic T lymphocyte responses to exogenous hepatitis B surface antigen is CD4+ T cell-dependent. J. Immunol. 163: 1880-1887.
41. Williams, N. A., Hirst, T.R., and Nashar, T.O. (1999) Immune modulation by the cholera-like enterotoxins: from adjuvant to therapeutic. Immunol. Today 20: 95-101.
42. Zhan, Y., and Cheers, C. (1998) Control of IL-12 and IFN-γ production in response to live or dead bacteria by TNF and other factors. J. Immunol. 161:1447-1453.

**Table 1. Archaeosomes prepared from polar lipids isolated in a biologically pure form from archaeobacteria induce CTL activity.**

| Effector:target | % lysis of target cells | |
|---|---|---|
| | PGP-O-CH₃ | PG |
| 100:1 | 32 | 14 |
| 33:1 | 22 | 5 |
| 11:1 | 17 | 5 |
| 3.3:1 | 8 | 3 |

The purified polar lipid archaetidyl glycerolphosphate-O-methyl (PGP-O-CH₃ from *H. salinarum*) or archaetidyl glycerol (PG from *N. magadii*) was used to make archaeosomes with encapsulated ovalbumin. BALB/c mice were immunized twice (s.c.), 3 weeks apart, with 20 µg ovalbumin entrapped in 0.6-0.8 mg lipid. Cytotoxic T cell (CTL) activity was measured 3 weeks post final vaccination, as % lysis of the target cells at various effector: target ratios (EG.7 data minus control EL-4 data).

**Table 2. Burden of L. monocytogenes or S. typhimurium in the livers and spleens of control mice and vaccinated mice on day 3 post-challenge with one or other pathogen.**

| Vaccine group¹ | Challenge pathogen | Log₁₀ ± SD CFU pathogen per organ² | |
|---|---|---|---|
| | | Liver | Spleen |
| Unvaccinated | *L. monocytogenes* | 3.90 ± 0.33 | 5.11 ± 0.22* |
| Encapsulated | " | <2.97 (1/5)^{†} | <2.09 (4/5)^{†} |
| lipopeptide³ | | | |
| Lipopeptide alone⁴ | " | 4.04 ± 0.28 | 4.67 ± 0.59* |
| Archaeosomes | " | 4.53 ± 0.51* | 5.40 ± 0.42* |
| alone | | | |
| Admixed | " | 4.53 ± 0.37* | 4.79 ± 0.24* |
| Unvaccinated | *S. typhimurium* | 5.27 ± 0.29 | 4.89 ± 0.27 |
| Encapsulated | " | 5.16 ± 0.19 | 5.01 ± 0.30 |
| lipopeptide³ | | | |

| | | | |
|---|---|---|---|
| ¹ mice (n= 4-6 per group) were vaccinated (s.c.) on days 0, 21, 42 and were challenged with bacteria (1.66 x 10³ CFU *L. monocytogenes* /mouse or 2.42 x 10³CFU *S. typhimurium*/mouse*,* given i.v.) 28 days after the final vaccination; ² bacterial burdens (Log₁₀ ± SD CFU/organ) were determined 3 days after the challenge; ³ 12.5 µg 20-mer lipopeptide encapsulated in 0.5 mg *M. smithii* archaeosomes; ⁴ 12.5 µg 20-mer lipopeptide in PBS ; ⁵ 0.5 mg empty *M. smithii* archaeosomes; ⁶12.5 µg 20-mer lipopeptide admixed with 0.5 mg preformed *M. smithii* archaeosomes; * bacterial burden significantly higher than in corresponding organs of mice immunized with encapsulated peptide (p<0.05 by Mann Whitney rank sum test); ^{†} numbers in parenthesis indicate the proportion of mice in the group that had no detectable level of CFU/indicated organ; if parenthesis is not shown, respective organs in all mice in the group had detectable CFU (i.e., infection); CFU, colony forming units; SD, standard deviation | | | |

**Table 3. Immunity to Listeria persists in animals vaccinated with archaeosome-encapsulated antigen.**

| Group | Log ₁₀ ±SD CFU *Listeria* /organ | | | | |
|---|---|---|---|---|---|
| | 3 months | | 5 months | | 10 months |
| | Liver | Spleen | Liver | Spleen | Spleen |
| Unvaccinated | 4.64 ± 0.66 | 5.49 ± 0.38 | 3.93 ± 0.97 | 4.75 ± 0.67 | 4.66 ± 0.32 |
| | | | | | |
| Vaccinated ^{¶} | <3.44 (1/5)^{†*} | <2.17 (4/5)^{†*} | <2.74 (1/5)^{†*} | <2.12 (4/5)^{†*} | <2.00 (1/5) |

| | | | | | |
|---|---|---|---|---|---|
| ^{¶} Mice (4-5 per group) were vaccinated as in Table 2, with 20-mer lipopeptide antigen encapsulated in *M. smithii* archaeosomes. After 3, 5, and 10 months subsequent to the final vaccination, vaccinated and unvaccinated (control) groups of mice were challenged with 10³ CFU *L. monocytogenes.* Bacterial burdens were determined (Log₁₀ ±SD CFU *Listeria* /organ) 3 days after the challenge. * significantly lower bacterial burden (p< 0.05) than in corresponding organ from the unvaccinated control group by the Mann Whitney rank sum test. ^{†} numbers in parenthesis indicate the proportion of mice in the group that had no detectable *Listeria*/indicated organ; if parenthesis is not shown, respective organs in all mice in the group had detectable CFU (i.e., infection). CFU, colony forming units; SD, standard deviation. | | | | | |

**Table 4. Early anti-Listeria immunity following vaccination with archaeosome-encapsulated lipopeptide antigen.**

| Vaccine group | Log₁₀ ± SD *Listeria*/organ | |
|---|---|---|
| | Liver | Spleen |
| Unvaccinated | 4.90 ± 0.54 | 5.69 ± 0.61 |
| *H. salinarum* archaeosomes, no antigen | 4.25 ± 0.50 | 5.09 ± 0.33 |
| *H. salinarum* archaeosomes, antigen incorporated | 2.66 ± 0.55*^{†} | 2.42 ± 0.44*^{†} |
| *M. smithii* archaeosomes, no antigen | 4.31 ± 0.46 | 5.20 ± 0.33 |
| *M. smithii* archaeosomes, antigen incorporated | 3.60 ± 0.86* | 2.73 ± 0.63*^{†} |

| | | |
|---|---|---|
| Mice (n = 4 or 5 per group) were vaccinated (s.c.) once with 15 µg of 20-mer lipopeptide entrapped in 0.6 mg of *M. smithii* or *H. salinarum* archaeosomes, or with empty archaeosomes. Seven days after vaccination, all mice were challenged with an i.v. inoculum of 2.5 x 10³ CFU of *L. monocytogenes*. Three days after the challenge, bacterial burdens (Log₁₀ ± SD *Listeria*/organ) in the livers and spleens were determined. * significantly lower bacterial burden (p<0.05) than in the corresponding organ in the unvaccinated control group. ^{†} significantly lower bacterial burden (p<0.05) in the corresponding organ than in mice immunized with the corresponding empty (no antigen) archaeosomes. | | |

**Table 5. Extent of early anti-Listeria immunity induced by vaccination with lipopeptide encapsulated in various vesicle types.**

| Delivery vehicle | Challenge pathogen | Log₁₀ CFU ± SD of the pathogen/orqan | |
|---|---|---|---|
| | | Liver | Spleen |
| *T. acidophilum* archaeosomes (0.37 mg)^{¶} | *L. monocytogenes* | 2.84 ± 0.76* | < 2.00 (4/5)^{†*} |
| *M. smithii* archaeosomes (0.67 mg)^{¶} | " | 3.70 ± 0.51* | 2.76 ± 0.08* |
| *H. salinarum* archaeosomes (0.60 mg)^{¶} | " | 3.01 ± 0.46* | 2.46 ± 0.51* |
| *N. magadii* archaeosomes (0.66 mg)^{¶} | " | 3.40 ± 0.22* | 2.81 ± 0.67* |
| Conventional liposomes (0.54 mg)^{¶} | " | 4.35 ± 0.84 | 4.03 ± 0.76* |
| Naïve controls | " | 4.58 ± 0.38 | 5.54 ± 0.73 |
| *H. salinarum* archaeosomes (0.60 mg)^{¶} | S. *typhimurium* | 5.73 ± 0.18 | 6.02 ± 0.14 |
| Naïve controls | " | 5.66 ± 0.08 | 5.95 ± 0.10 |

| | | | |
|---|---|---|---|
| Mice (n= 5 per group) were vaccinated once with 15 µg of the 20-mer lipopeptide encapsulated in the stated ( )^{¶} quantity of one or another vesicle type. One week post-vaccination, mice were challenged intravenously with either *L. monocytogenes* (2.5 x 10³ CFU/ mouse) or S. *typhimurium* (3.1 x 10³ CFU/ mouse). Bacterial burdens (Log₁₀ CFU ± SD of the pathogenlorgan) in the livers and spleens were determined 3 days after the challenge. * bacterial burden significantly lower (p<0.05) than that in corresponding organs of naïve control group by Mann Whitney rank sum test; ^{†} numbers in parenthesis indicate the proportion of mice in the group that had no detectable CFU pathogen/indicated organ; if parenthesis is not shown, respective organs in all mice in the group had detectable CFU (i.e., infection). | | | |

**Table 6. Specificity of early immunity induced by vaccination with antigen encapsulated in T. acidophilum archaeosomes.**

| | | | | |
|---|---|---|---|---|
| Treatment | Mouse strain | Challenge pathogen | Log₁₀ ±SD CFU pathogen/ organ | |
| | | | Liver | Spleen |
| | | | | |
| Vaccinated¹ | BALB/c | *L. monocytogenes* | <2.18 (3/5)^{†*} | <2.00 (4/5)^{†*} |
| Naïve | BALB/c | " | 4.29± 0.62 | 5.17 ±0.70 |
| Vaccinated¹ | C57BU6 | " | 3.47± 0.64 | 4.67 ±0.39 |
| Naïve | C57BU6 | " | 3.81± 0.37 | 4.96 ±0.16 |
| Vaccinated¹ | BALB/c | *S. typhimurium* | 5.64 ±0.19 | 5.89 ±0.14 |
| Naïve | BALB/c | " | 5.76 ±0.19 | 6.01 ±0.20 |

| | | | | |
|---|---|---|---|---|
| ¹Mice were vaccinated once with the 20-mer lipopeptide (11 µg) encapsulated in T. *acidophilum* archaeosomes (0.28 mg), then challenged 7 days later with either *L. monocytogenes* (2.23 x 10³ CFU/ mouse) or S. *typhimurium* (3.61 x 10³ CFU/mouse). Bacterial burdens (Log₁₀ ±SD CFU pathogen/ organ) in the liver and spleen were determined 3 days after the challenge. * bacterial burden significantly lower (p<0.05) than in corresponding organ of the naive (unvaccinated) groups of mice. ^{†} numbers in parenthesis indicate the proportion of mice in the group that had no detectable level of CFU pathogen/indicated organ; if parenthesis is not shown, respective organs in all mice in the group had detectable CFU (i.e., infection). | | | | |

**Table 7. Ability of various archaeosomal vaccines to elicit prolonged anti-Listeria immunity.**

| Delivery vehicle | Log₁₀ CFU ± SD of *Listeria* /organ | |
|---|---|---|
| | Liver | Spleen |
| *T. acidophilum* archaeosomes | 3.71 ±0.42* | 3.52 ±0.62* |
| *M. smithii* archaeosomes | 3.82 ±0.28* | 3.52 ±0.62* |
| *H. salinarum* archaeosomes | 3.60 ±0.38* | 3.77 ±0.30* |
| Naïve controls | 4.89 ±0.58 | 5.69 ±0.61 |

| | | |
|---|---|---|
| Mice (n=5 per group) were vaccinated once with 15 µg of the 20-mer lipopeptide encapsulated in one or another archaeosome (archaeosome quantities as stated in Table 5). One month post-vaccination, mice were challenged intravenously with *L. monocytogenes* (2.5 x 10³ CFU/ mouse). Bacterial burdens (Log₁₀ CFU ± SD of *Listeria* /organ) in the livers and spleens were determined 3 days after the challenge. * bacterial burden significantly lower (p<0.05) than that in the corresponding organs of the naive (unvaccinated) control mice group by Mann Whitney rank sum test. | | |

**Table 8. Ability of lipopeptide antigen entrapped in various vesicle types to elicit prolonged anti-Listeria immunity, when challenged with a large inoculum of the pathogen.**

| Delivery vehicle | Log₁₀ CFU ± SD *Listeria* /organ | |
|---|---|---|
| | Liver | Spleen |
| | | |
| *T. acidophilum* archaeosomes | 3.62 ±0.40* | <2.54 (3/4) ^{†*} |
| *M. smithii* archaeosomes | <2.74 (1/3)^{†*} | <2.00 (3/3) ^{†*} |
| *H. salinarum* archaeosomes | 3.35± 0.87* | 4.23 ±1.22* |
| Conventional liposomes | 4.48 ±0.44* | 4.18 ±1.04* |
| *M. smithii* archaeosomes, no | 6.21 ±1.12 | 6.26 ±0.33 |
| antigen | | |

| | | |
|---|---|---|
| Mice (n=3 or 4 per group) were vaccinated twice, 28 days apart, with 15 µg of the 20-mer lipopeptide encapsulated in one or another vehicle type (quantities stated in Table 5), or with empty *M. smithii* archaeosomes (no antigen). One month post-vaccination, mice were challenged intravenously with *L. monocytogenes* (2.0 x 10⁴ CFU/ mouse). Bacterial burdens (Log₁₀ CFU ± SD *Listeria* /organ) in the livers and spleens were determined 3 days after the challenge. * bacterial burden significantly lower (P<0.05) than in corresponding organs of *M. smithii* archaeosomes, no antigen control group by Mann Whitney rank sum test. ^{†} numbers in parenthesis indicate the proportion of mice in the group that had no detectable level of CFU *Listeria* /indicated organ; if parenthesis is not shown, respective organs in all mice in the group had detectable CFU (i.e., infection). | | |

**Table 9. Rapid onset of immunity against infection by Listeria monocytogenes in BALB/c mice immunized with decreasing amounts of the 13-mer lipopeptide encapsulated in T. acidophilum archaeosomes.**

| µg archaeosomes | µg 13-mer lipopeptide | Log₁₀ CFU ± SD of *Listeria* /organ | |
|---|---|---|---|
| | | Spleen | Liver |
| 500 | 23.0 | <2.971 (1/5) ^{†} | <2.843 (1/5) ^{†} |
| 250 | 11.5 | <2.389 (3/5) ^{†} | <3.374 (3/5) ^{†} |
| 50 | 2.3 | <2.00 (5/5) ^{†} | 2.914 ±0.31 |
| 10 | 0.5 | <2.699(4/5) ^{†} | <3.341 (1/5) ^{†} |
| 0 | 0.0 | 5.928 ±0.22 | 4.827 ±0.19 |

| | | | |
|---|---|---|---|
| Mice (5 animals/group) were vaccinated once (s.c.) with archaeosomes containing 13-mer lipopeptide at the concentrations shown in the table. At 1 week post-vaccination, the animals were challenged (i.v.) with 1.26 x 10⁴ CFU of *L. monocytogenes,* and the bacterial burdens (Log₁₀ CFU ± SD of *Listeria* /organ) in spleens and livers determined 3 days after the challenge. ^{†} numbers in parenthesis indicate the proportion of mice in the group that had no detectable level of CFU of *Listeria*/indicated organ; if parenthesis is not shown, respective organs in all mice in the group had detectable CFU (i.e., infection). | | | |

**Table 10. Immunity to infection by an aerosol challenge of Francisella tularensis LVS in BALB/c mice immunized with M. smithii archaeosomes fused with isolated outer membrane preparation from F. tularensis.**

| Treatment | | Log₁₀ CFU ± SD | |
|---|---|---|---|
| | Lung | Spleen | Liver |
| Naïve | 6.85 ±0.15 | 4.07 ±0.39 | 4.27 ±0.25 |
| Immunized | 5.27 ±0.13 | <2.77 (1/6) ^{†} | <3.90 (1/6) ^{†} |

| | | | |
|---|---|---|---|
| Mice (6 animals/group) were vaccinated subcutaneously, at the base of the tail, at 0, 3 and 5 weeks, with 1 mg *M. smithii* archaeosomes containing the outer membrane (1.6 µg protein) extracted from *F. tularensis.* At 7 weeks post first vaccination, animals were challenged by aerosol (for 5 min) with a suspension of *F. tularensis* LVS containing 6.9x10⁹ CFU per ml. The bacterial burdens (Log₁₀ CFU ±SD of *F. tularensis* /organ) in lungs, spleens and livers were determined by plate count 3 days after the challenge. ^{†} numbers in parenthesis indicate the proportion of mice in the group that had no detectable level of CFU of *Francisella tularensis* /indicated organ; if parenthesis is not shown, respective organs in all mice in the group had detectable CFU (i.e., infection). | | | |

## Claims

1. Use of a composition comprising a liposome prepared from a polar lipid extract of an archeobacterium and an antigen having an MHC class I restricted epitope in the manufacture of a medicament for prophylactic and/or therapeutic use against cancer or infection by intracellular pathogens, wherein the liposome serves as an adjuvant, and the medicament elicits an antigen-specific CD8* T cell response in an animal.

2. Use of a composition according to claim 1, wherein the medicament is a vaccine.

3. Use of a composition according to Claim 1 or 2, wherein the liposome serves as an immunomodulating carrier for the antigen.

4. Use of a composition according to any of Claim 1 to 3, wherein the polar lipid extract is the total polar lipids extract of an archaeobacterium.

5. Use of a composition according to Claims 1 or 2, wherein the polar lipid extract is archaetidyl glycerollphosphate-O-methyl.

6. Use of a composition according to any one of the previous Claims, wherein the elicited antigen-specific CD8⁺ T cell response is a cytotoxic T cell response.

7. Use of a composition according to any one of the previous Claims, wherein the medicament is administered to an animal by the parenteral route.

8. Use of a composition according to any one of the previous Claims, wherein the antigen is an acellular antigen, a peptide, an alkylated peptide amino acid sequence, a protein or an outer membrane preparation isolated from a pathogen.

9. Use of a composition according to Claims 1 to 4, wherein the CD8⁺ T cell response elicited in the animal promotes a CD8⁺ T cell memory response.

10. Use of a composition according to Claims 1 to 4, wherein re-exposure of the animal to the antigen upregulates the expression of CD44 memory marker on T cells.

11. Use of a composition according to Claims 1 to 4, wherein the archaeobacterium is selected from the group comprising *Methanobrevibacter smithii, Thermoplasma acidophilum,* or *Halobacterium salinarum.*

12. Use of a composition according to Claim 11, wherein the archaeobacterium is *Methanobrevibacter smithii.*

13. Use of a composition according to Claim 11, wherein the archaeobacterium is *Thermoplasma acidophilum.*

14. Use of a composition according to Claim 11, wherein the archaeobacterium is *Halobacterium salinarum.*

15. Use of a composition according to Claim 12, wherein the antigen-specific CD8⁺ T cell response in the animal is elicited in the absence of CD4⁺ T cell help.

16. Use of a composition according to Claim 12, wherein the generation of an antigen-specific CD8⁺ T cell response is mediated through activation of antigen presenting cells by the liposome adjuvant.

17. Use of a composition according to Claim 16, wherein the activation of antigen presenting cells occurs via upregulation of costimulatory molecules B7.1 (CD80) and B7.2 (CD86) on the surface of the antigen presenting cells.

18. Use of a composition according to Claim 16 or 17, wherein the antigen presenting cells activated are the CD11c⁺ dendritic cells.

19. Use of composition according to claim 12, wherein the generation of an antigen-specific CD8⁺ T cell response results in the production of the cytokine interferon gamma by the CD8+ T cells.

20. Use of composition according to claims 1-4, wherein the generation of an antigen specific T cell response additionally results in the stimulation of the production of cytokine IL-4 by T cells.

21. Use of a composition according to Claim 12, wherein the liposome adjuvant stimulates the production of tumor necrosis factor by antigen presenting cells.

22. Use of a composition according to Claim 16, wherein the antigen presenting cells activated are the Mac 1α^{hi} cells.

23. Use of a composition according to Claims 1 to 4, for conferring to an animal protective immunity against infection by an intracellular pathogen.

24. Use of a composition according to Claim 23, wherein the intracellular pathogen is a virus, a bacterium or a parasite.

25. Use of a composition according to Claim 23 or 24, wherein the antigen is an alkylated peptide amino acid sequence corresponding to an amino acid sequence expressed by the pathogen.

26. Use of a composition according to Claim 23 or 24, wherein the antigen is an isolated outer membrane preparation from the pathogen.

27. Use of a composition according to Claim 23, wherein the dosage of the vaccine composition comprises 10 to 670 µg of liposomes and 0.5 to 23 µg of antigen.

28. Use of a composition according to Claim 27, wherein the dosage of the vaccines composition comprises 10 µg of liposomes and 0.5 µg of antigen.

29. Use of a composition according to Claim 23, wherein the protective immunity conferred to the animal is specific for the antigen.

30. Use of a composition according to Claim 23, wherein a single subcutaneous injection of the vaccine composition to an animal is sufficient to confer protective immunity to the animal.

31. Use of a composition according to Claim 30, wherein the onset of protective immunity occurs within 7 days of a single, subcutaneous administration of the vaccine composition to the animal.

32. Use of a composition according to Claim 30 or 31, wherein the protective immunity is observed in the vaccinated animal within 24 to 48 hours after an infectious challenge.

33. Use of a composition according to Claim 23, wherein the archaeobacterium is *Thermoplasma acidophilum, Methanobrevibacter smithii, Natronobacterium magadii,* or *Halobacterium salinarum.*

34. Use of a composition according to Claim 23, wherein the pathogen is *Francisella tularensis.*

35. Use of a composition according to Claim 34, ***characterised in that*** the antigen is an isolated outer membrane preparation from *Francisella tularensis.*

36. Use of a composition according to Claim 23, ***characterised in that*** the pathogen is *Listeria monocytogenes.*

37. Use of a composition according to Claim 36, ***characterised in that*** the dosage of the vaccine composition comprises 10 to 500 µg of liposomes prepared from the total polar lipids extract from *Thermoplasma acidophilum* and 0.5 to 23 µg of the antigen.

38. Use of a composition according to Claim 23, ***characterised in that*** the conferred protective immunity can elicit a memory response.

39. Use of a composition according to Claim 38, ***characterised in that*** the conferred memory response confers to the animal protective immunity over a significant portion of the life span of the animal.

40. Use of a composition according to Claim 4, ***characterised in that*** CD4⁺ Th1 and Th2 responses are additionally induced in an animal.

41. Use of a composition according to Claim 40, ***characterised in that*** an antigen-specific CD4⁺ T cell and an antigen-specific CD8⁺ T cell memory response is elicited in the animal.

42. Use of a composition according to Claim 41, ***characterised in that*** the archaeobacterium is *Thermoplasma acidophilum.*

43. Use of a composition according to Claims 1 to 4, for conferring to an animal protective immunity against cancer.

44. Use of a composition according to Claims 1 to 4, for conferring to an animal therapeutic immunity against cancer.

45. Use of a composition according to Claim 43, ***characterised in that*** the antigen in the vaccine composition is expressed by the cancer cell.

## Patentansprüche

1. Eine Verwendung einer Zusammensetzung, die ein Liposom, das aus einem Extrakt aus polarem Lipid eines Archaebacteriums zubereitet wurde, und ein Antigen mit einem MHC-Klasse I restringierten Epitop beinhaltet, bei der Herstellung eines Medikaments für die prophylaktische und/oder therapeutische Verwendung gegen Krebs oder gegen eine Infektion durch intrazelluläre Pathogene, wobei das Liposom als Adjuvans dient und das Medikament in einem Tier eine antigenspezifische CD8⁺-T-Zell-Antwort hervorruft.

2. Verwendung einer Zusammensetzung gemäß Anspruch 1, wobei das Medikament ein Impfstoff ist.

3. Verwendung einer Zusammensetzung gemäß Anspruch 1 oder 2, wobei das Liposom als immunmodulierender Träger für das Antigen dient.

4. Verwendung einer Zusammensetzung gemäß einem der Ansprüche 1 bis 3, wobei der Extrakt aus polarem Lipid der Extrakt aus den gesamten polaren Lipiden eines Archaebacteriums ist.

5. Verwendung einer Zusammensetzung gemäß Anspruch 1 oder 2, wobei der Extrakt aus polarem Lipid Archaetidylglyzerinphosphat-O-methyl ist.

6. Verwendung einer Zusammensetzung gemäß einem der vorhergehenden Ansprüche, wobei die hervorgerufene antigenspezifische CD8⁺-T-Zell-Antwort eine zytotoxische T-Zell-Antwort ist.

7. Verwendung einer Zusammensetzung gemäß einem der vorhergehenden Ansprüche, wobei das Medikament einem Tier auf dem parenteralen Weg verabreicht wird.

8. Verwendung einer Zusammensetzung gemäß einem der vorhergehenden Ansprüche, wobei das Antigen ein azelluläres Antigen, ein Peptid, eine Aminosäuresequenz eines alkylierten Peptids, ein Protein oder ein Präparat einer Außenmembran, die von einem Pathogen isoliert wurde, ist.

9. Verwendung einer Zusammensetzung gemäß den Ansprüchen 1 bis 4, wobei die CD8⁺-T-Zell-Antwort, die in dem Tier hervorgerufen wird, eine CD8⁺-T-Zell-Gedächtnisantwort fördert.

10. Verwendung einer Zusammensetzung gemäß den Ansprüchen 1 bis 4, wobei die Expression des CD44-Gedächtnismarkers auf T-Zellen hochreguliert wird, wenn das Tier dem Antigen erneut ausgesetzt wird.

11. Verwendung einer Zusammensetzung gemäß den Ansprüchen 1 bis 4, wobei das Archaebacterium aus der Gruppe ausgewählt wird, die *Methanobrevibacter smithii, Thermoplasma acidophilum* oder *Halobacterium salinarum* beinhaltet.

12. Verwendung einer Zusammensetzung gemäß Anspruch 11, wobei das Archaebacterium *Methanobrevibacter smithii* ist.

13. Verwendung einer Zusammensetzung gemäß Anspruch 11, wobei das Archaebacterium *Thermoplasma acidophilum* ist.

14. Verwendung einer Zusammensetzung gemäß Anspruch 11, wobei das Archaebacterium *Halobacterium salinarum* ist.

15. Verwendung einer Zusammensetzung gemäß Anspruch 12, wobei die antigenspezifische CD8⁺-T-Zell-Antwort in dem Tier in der Abwesenheit von Hilfe durch CD4⁺-T-Zellen hervorgerufen wird.

16. Verwendung einer Zusammensetzung gemäß Anspruch 12, wobei die Erzeugung einer antigenspezifischen CD8⁺-T-Zell-Antwort durch die Aktivierung von Antigen präsentierenden Zellen durch das Liposomadjuvans vermittelt wird.

17. Verwendung einer Zusammensetzung gemäß Anspruch 16, wobei die Aktivierung von Antigen präsentierenden Zellen über die Hochregulierung von kostimulierenden Molekülen B7.1 (CD80) und B7.2 (CD86) auf der Oberfläche der Antigen präsentierenden Zellen erfolgt.

18. Verwendung einer Zusammensetzung gemäß Anspruch 16 oder 17, wobei die aktivierten Antigen präsentierenden Zellen die dendritischen CD11c⁺-Zellen sind.

19. Verwendung einer Zusammensetzung gemäß Anspruch 12, wobei die Erzeugung einer antigenspezifischen CD8⁺-T-Zell-Antwort in der Produktion des Zytokins Gamma-Interferon durch die CD8⁺-T-Zellen resultiert.

20. Verwendung einer Zusammensetzung gemäß den Ansprüchen 1-4, wobei die Erzeugung einer antigenspezifischen T-Zell-Antwort zusätzlich dazu in der Stimulierung der Produktion des Zytokins IL-4 durch T-Zellen resultiert.

21. Verwendung einer Zusammensetzung gemäß Anspruch 12, wobei das Liposomadjuvans die Produktion des Tumor-Nekrose-Faktors durch Antigen präsentierende Zellen stimuliert.

22. Verwendung einer Zusammensetzung gemäß Anspruch 16, wobei die aktivierten Antigen präsentierenden Zellen Mac-1α^{hi}-Zellen sind.

23. Verwendung einer Zusammensetzung gemäß den Ansprüchen 1 bis 4, um einem Tier eine schützende Immunität gegen die Infektion durch ein intrazelluläres Pathogen zu verleihen.

24. Verwendung einer Zusammensetzung gemäß Anspruch 23, wobei das intrazelluläre Pathogen ein Virus, ein Bakterium oder ein Parasit ist.

25. Verwendung einer Zusammensetzung gemäß Anspruch 23 oder 24, wobei das Antigen eine Aminosäuresequenz eines alkylierten Peptids ist, die einer von dem Pathogen exprimierten Aminosäuresequenz entspricht.

26. Verwendung einer Zusammensetzung gemäß Anspruch 23 oder 24, wobei das Antigen ein Präparat einer isolierten Außenmembran des Pathogens ist.

27. Verwendung einer Zusammensetzung gemäß Anspruch 23, wobei die Dosierung der Impfstoffzusammensetzung 10 bis 670 µg Liposome und 0,5 bis 23 µg Antigen beinhaltet.

28. Verwendung einer Zusammensetzung gemäß Anspruch 27, wobei die Dosierung der Impfstoffzusammensetzung 10 µg Liposome und 0,5 µg Antigen beinhaltet.

29. Verwendung einer Zusammensetzung gemäß Anspruch 23, wobei die dem Tier verliehene schützende Immunität spezifisch für das Antigen ist.

30. Verwendung einer Zusammensetzung gemäß Anspruch 23, wobei eine einzelne subkutane Injektion der Impfstoffzusammensetzung in ein Tier ausreicht, um dem Tier eine schützende Immunität zu verleihen.

31. Verwendung einer Zusammensetzung gemäß Anspruch 30, wobei der Beginn der schützenden Immunität innerhalb von 7 Tagen nach einer einzelnen, subkutanen Verabreichung der Impfstoffzusammensetzung an das Tier erfolgt.

32. Verwendung einer Zusammensetzung gemäß Anspruch 30 oder 31, wobei die schützende Immunität innerhalb von 24 bis 48 Stunden nach einem infektiösen Angriff in dem geimpften Tier beobachtet wird.

33. Verwendung einer Zusammensetzung gemäß Anspruch 23, wobei das Archaebacterium *Thermoplasma acidophilum, Methanobrevibacter smithii, Natronobacterium magadii* oder *Halobacterium salinarum* ist.

34. Verwendung einer Zusammensetzung gemäß Anspruch 23, wobei das Pathogen *Francisella tularensis* ist.

35. Verwendung einer Zusammensetzung gemäß Anspruch 34, **dadurch gekennzeichnet, dass** das Antigen ein Präparat einer isolierten Außenmembran von *Francisella tularensis* ist.

36. Verwendung einer Zusammensetzung gemäß Anspruch 23, **dadurch gekennzeichnet, dass** das Pathogen *Listeria monocytogenes* ist.

37. Verwendung einer Zusammensetzung gemäß Anspruch 36, **dadurch gekennzeichnet, dass** die Dosierung der Impfstoffzusammensetzung 10 bis 500 µg Liposome, die aus dem Extrakt aus den gesamten polaren Lipiden von *Thermoplasma acidophilum* zubereitet wurden, und 0,5 bis 23 µg Antigen beinhaltet.

38. Verwendung einer Zusammensetzung gemäß Anspruch 23, **dadurch gekennzeichnet, dass** die verliehene schützende Immunität eine Gedächtnisantwort hervorrufen kann.

39. Verwendung einer Zusammensetzung gemäß Anspruch 38, **dadurch gekennzeichnet, dass** die verliehene Gedächtnisantwort dem Tier über einen signifikanten Anteil der Lebensdauer des Tieres eine schützende Immunität verleiht.

40. Verwendung einer Zusammensetzung gemäß Anspruch 4, **dadurch gekennzeichnet, dass** zusätzlich dazu in einem Tier CD4⁺-Th1 und -Th2-Antworten induziert werden.

41. Verwendung einer Zusammensetzung gemäß Anspruch 40, **dadurch gekennzeichnet, dass** eine antigenspezifische Gedächtnisantwort von CD4⁺-T-Zellen und eine antigenspezifische Gedächtnisantwort von CD8⁺-T-Zellen in dem Tier hervorgerufen werden.

42. Verwendung einer Zusammensetzung gemäß Anspruch 41, **dadurch gekennzeichnet, dass** das Archaebacterium *Thermoplasma acidophilum* ist.

43. Verwendung einer Zusammensetzung gemäß den Ansprüchen 1 bis 4, um einem Tier eine schützende Immunität gegen Krebs zu verleihen.

44. Verwendung einer Zusammensetzung gemäß den Ansprüchen 1 bis 4, um einem Tier eine therapeutische Immunität gegen Krebs zu verleihen.

45. Verwendung einer Zusammensetzung gemäß Anspruch 43, **dadurch gekennzeichnet, dass** das Antigen in der Impfstoffzusammensetzung von der Krebszelle exprimiert wird.

## Revendications

1. Utilisation d'une composition comprenant un liposome préparé à partir d'un extrait de lipide polaire d'une archaebactérie et un antigène ayant un épitope restreint à la molécule CMH de classe I dans la fabrication d'un médicament destiné à une utilisation prophylactique et / ou thérapeutique contre un cancer ou une infection par des agents pathogènes intracellulaires, où le liposome sert d'adjuvant, et le médicament sollicite une réponse de cellule T CD8⁺ spécifique à l'antigène chez un animal.

2. Utilisation d'une composition selon la revendication 1, où le médicament est un vaccin.

3. Utilisation d'une composition selon la revendication 1 ou 2, où le liposome sert de porteur immunomodulateur pour l'antigène.

4. Utilisation d'une composition selon n'importe lesquelles des revendications 1 à 3, où l'extrait de lipide polaire est l'extrait total de lipides polaires d'une archaebactérie.

5. Utilisation d'une composition selon la revendication 1 ou 2, où l'extrait de lipide polaire est de l'archaetidyle glycérolphosphate-O-méthyle.

6. Utilisation d'une composition selon n'importe laquelle des revendications précédentes, où la réponse sollicitée de cellule T CD8⁺ spécifique à l'antigène est une réponse de cellule T cytotoxique.

7. Utilisation d'une composition selon n'importe laquelle des revendications précédentes, où le médicament est administré à un animal par voie parentérale.

8. Utilisation d'une composition selon n'importe laquelle des revendications précédentes, où l'antigène est un antigène acellulaire, un peptide, une séquence d'acides aminés de peptide alkylé, une protéine ou une préparation de membrane externe isolée d'un agent pathogène.

9. Utilisation d'une composition selon les revendications 1 à 4, où la réponse de cellule T CD8⁺ sollicitée chez l'animal encourage une réponse mémoire de cellule T CD8⁺.

10. Utilisation d'une composition selon les revendications 1 à 4, où une ré-exposition de l'animal à l'antigène régule à la hausse l'expression de marqueur de mémoire CD44 sur des cellules T.

11. Utilisation d'une composition selon les revendications 1 à 4, où l'archaebactérie est sélectionnée dans le groupe comprenant *Methanobrevibacter smithii, Thermoplasma acidophilum,* ou *Halobacterium salinarum.*

12. Utilisation d'une composition selon la revendication 11, où l'archaebactérie est *Methanobrevibacter smithii.*

13. Utilisation d'une composition selon la revendication 11, où l'archaebactérie est *Thermoplasma acidophilum.*

14. Utilisation d'une composition selon la revendication 11, où l'archaebactérie est *Halobacterium salinarum.*

15. Utilisation d'une composition selon la revendication 12, où la réponse de cellule T CD8⁺ spécifique à l'antigène chez l'animal est sollicitée en l'absence d'aide de cellule T CD4⁺.

16. Utilisation d'une composition selon la revendication 12, où la génération d'une réponse de cellule T CD8⁺ spécifique à l'antigène est médiée par l'intermédiaire de l'activation de cellules présentant l'antigène par l'adjuvant de liposome.

17. Utilisation d'une composition selon la revendication 16, où l'activation de cellules présentant l'antigène a lieu par le biais d'une régulation à la hausse des molécules costimulantes B7.1 (CD80) et B7.2 (CD86) sur la surface des cellules présentant l'antigène.

18. Utilisation d'une composition selon la revendication 16 ou 17, où les cellules présentant l'antigène activées sont les cellules dendritiques CD11c⁺.

19. Utilisation d'une composition selon la revendication 12, où la génération d'une réponse de cellule T CD8⁺ spécifique à l'antigène a pour résultat la production de l'interféron gamma cytokine par les cellules T CD8⁺.

20. Utilisation d'une composition selon les revendications 1 à 4, où la génération d'une réponse de cellule T spécifique à l'antigène a pour résultat de façon additionnelle la stimulation de la production de cytokine IL-4 par les cellules T.

21. Utilisation d'une composition selon la revendication 12, où l'adjuvant de liposome stimule la production d'un facteur de nécrose tumorale par les cellules présentant l'antigène.

22. Utilisation d'une composition selon la revendication 16, où les cellules présentant l'antigène activées sont les cellules Mac 1α^{hi}.

23. Utilisation d'une composition selon les revendications 1 à 4, afin de conférer à un animal une immunité protectrice contre une infection par un agent pathogène intracellulaire.

24. Utilisation d'une composition selon la revendication 23, où l'agent pathogène intracellulaire est un virus, une bactérie ou un parasite.

25. Utilisation d'une composition selon la revendication 23 ou 24, où l'antigène est une séquence d'acides aminés de peptide alkylé correspondant à une séquence d'acides aminés exprimée par l'agent pathogène.

26. Utilisation d'une composition selon la revendication 23 ou 24, où l'antigène est une préparation de membrane externe isolée provenant de l'agent pathogène.

27. Utilisation d'une composition selon la revendication 23, où le dosage de la composition de vaccin comprend de 10 à 670 µg de liposomes et de 0,5 à 23 µg d'antigène.

28. Utilisation d'une composition selon la revendication 27, où le dosage de la composition de vaccin comprend 10 µg de liposomes et 0,5 µg d'antigène.

29. Utilisation d'une composition selon la revendication 23, où l'immunité protectrice conférée à l'animal est spécifique pour l'antigène.

30. Utilisation d'une composition selon la revendication 23, où une injection sous-cutanée unique de la composition de vaccin à un animal est suffisante pour conférer une immunité protectrice à l'animal.

31. Utilisation d'une composition selon la revendication 30, où le début de l'immunité protectrice se produit sous 7 jours suivant une administration sous-cutanée unique de la composition de vaccin à l'animal.

32. Utilisation d'une composition selon la revendication 30 ou 31, où l'immunité protectrice est observée chez l'animal vacciné sous 24 à 48 heures après un test de provocation d'infection.

33. Utilisation d'une composition selon la revendication 23, où l'archaebactérie est *Thermoplasma acidophilum, Methanobrevibacter smithii, Natronobacterium magadii,* ou *Halobacterium salinarum.*

34. Utilisation d'une composition selon la revendication 23, où l'agent pathogène est *Francisella tularensis.*

35. Utilisation d'une composition selon la revendication 34, **caractérisée en ce que** l'antigène est une préparation de membrane externe isolée provenant de *Francisella tularensis.*

36. Utilisation d'une composition selon la revendication 23, **caractérisée en ce que** l'agent pathogène est *Listeria monocytogenes.*

37. Utilisation d'une composition selon la revendication 36, **caractérisée en ce que** le dosage de la composition de vaccin comprend de 10 à 500 µg de liposomes préparés à partir de l'extrait total de lipides polaires de *Thermoplasma acidophilum* et de 0,5 à 23 µg de l'antigène.

38. Utilisation d'une composition selon la revendication 23, **caractérisée en ce que** l'immunité protectrice conférée peut solliciter une réponse mémoire.

39. Utilisation d'une composition selon la revendication 38, **caractérisée en ce que** la réponse mémoire conférée confère à l'animal une immunité protectrice sur une portion significative de la durée de vie de l'animal.

40. Utilisation d'une composition selon la revendication 4, **caractérisée en ce que** des réponses CD4⁺ Th1 et Th2 sont induites de façon additionnelle chez un animal.

41. Utilisation d'une composition selon la revendication 40, **caractérisée en ce qu'**une cellule T CD4⁺ spécifique à l'antigène et une réponse mémoire de cellule T CD8⁺ spécifique à l'antigène sont sollicitées chez l'animal.

42. Utilisation d'une composition selon la revendication 41, **caractérisée en ce que** l'archaebactérie est *Thermoplasma acidophilum.*

43. Utilisation d'une composition selon les revendications 1 à 4, afin de conférer à un animal une immunité protectrice contre un cancer.

44. Utilisation d'une composition selon les revendications 1 à 4, afin de conférer à un animal une immunité thérapeutique contre un cancer.

45. Utilisation d'une composition selon la revendication 43, **caractérisée en ce que** l'antigène dans la composition de vaccin est exprimé par la cellule cancéreuse.
